Europäisches Patentamt

European Patent Office

Office européen des brevets

(19)

(11) Publication number: **0 359 998 B1**

(12) **EUROPEAN PATENT SPECIFICATION**

(45) Date of publication of patent specification: **18.05.94**

(51) Int. Cl.⁵: **C12N 15/12**, C12P 21/00, C12Q 1/70, C12P 21/02

(21) Application number: **89115158.1**

(22) Date of filing: **17.08.89**

Divisional application 93105511.5 filed on 17/08/89.

(54) **Factor regulating gene expression.**

(30) Priority: **24.08.88 EP 88113793**
**24.11.88 EP 88119602**

(43) Date of publication of application:
**28.03.90 Bulletin 90/13**

(45) Publication of the grant of the patent:
**18.05.94 Bulletin 94/20**

(84) Designated Contracting States:
**AT BE CH DE ES FR GB GR IT LI LU NL SE**

(56) References cited:

**Cell, vol. 49, 8 May 1987, pages 357-367, Cell Press, Cambridge, US, T. Fujita et al "Interferon-beta gene regulation : Tandemly repeated sequences of a synthetic 6 bp oligomer function as a virum-inducible anhancer"**

**Proceedings of the National Academy of Science, USA, vol. 85, May 1988, pages 3309-3313, A.D. Keller et al "Identification of an inducible factor that binds to a positive regulatory element of the human beta-interferon gene"**

(73) Proprietor: **Taniguchi, Tadatsugu, Prof. Dr.**
**Mihogaoka 19, A-207**
**Ibaraki-shi**
**Osaka 567(JP)**

(72) Inventor: **Taniguchi, Tadatsugu, Dr.**
**Mihogaoka 19, A-207**
**Ibaraki-shi**
**Osaka 567(JP)**
Inventor: **Fujita, Takashi, Dr.**
**Aogein 7-140**
**Ninov-shi**
**Osaka 562(JP)**

(74) Representative: **Laudien, Dieter, Dr. et al**
**Boehringer Ingelheim Zentrale GmbH**
**ZA Patente**
**Postfach 200**
**D-55216 Ingelheim am Rhein (DE)**

EP 0 359 998 B1

Annual Reviews of Immunology, vol. 6, 1988, pages 439-464, Annual Reviews Inc. Palo Alto, US, T. Taniguchi "Regulation of cytokine gene expression"

Cell, vol. 54, September 9, 1988, pages 903-913, Cell Press, Cambridge, US, M. Miyamoto et al "Regulated expression of a gene encoding a nuclear factor, IRF-1, that specifically binds to IFN-beta gene regulatory elements"

**Description**

The present invention relates generally to the regulation of gene expression. In particular the invention relates to a recombinant DNA molecule coding for protein having the activity of an interferon regulatory factor-1 (IRF-1); to recombinant DNA molecules characterized by a DNA sequence coding for an IRF-1 active protein and a promoter and regulator sequence operably linked thereto; to the use of such DNA molecules for transforming host cells which are also transformed by DNA molecules coding for a desired protein and under the control of said IRF-I active protein; to such DNA molecules including a sequence coding for a pharmaceutically active protein and a promoter region of the gene for said protein including a binding site for the IRF-1 active molecule; and to the production of said IRF-1 active protein and/or said pharmaceutically active protein by cultivation of suitable host cells transformed by said DNA molecules.

Transcription of genes in mammalian cells is regulated by complex mechanisms wherein interactions of the regulatory DNA sequences with trans-acting DNA binding proteins play a central role. In the context of the regulation of transcription, genes encoding interferons (IFNs) represent a feature common to many of the cytokine genes; transcription of those genes is induced in a transient manner following various extracellular signals. It has been well documented that transcription of the genes for IFN-$\alpha$ and IFN-$\beta$ is efficiently induced by viruses in a variety of cells, while that of the gene encoding IFN-$\gamma$ is induced in T lymphocytes (T cells) following mitogenic stimulation (for review see Weissmann and Weber, 1986; Taniguchi, 1988).

IFN-$\beta$, a cytokine that was originally identified for its potent antiviral activity, also appears to play a crucial role in controlling cell growth and differentiation. In this regard, beside viruses and poly(rl):poly(rC) which are the well known inducers of IFN-$\beta$ gene, many of the cytokines such as colony stimulating factor-1 (CSF-1) (Moore et al., 1984; Warren and Ralf, 1986; Resnitzky et al., 1986), tumor necrosis factor (TNF) (Onozaki et al., 1988), platelet-derived growth factor (PDGF) (Zullo et al., 1985) and IFNs (Kohase et al., 1987) also appear to induce IFN-$\beta$ in certain cells, suggesting that they may transduce similar or identical signals in the target cells.

The IFN-$\beta$ gene induction by viruses and poly(rl):poly(rC) has been shown to occur at the transcriptional level (Raji and Pitha, 1983; Ohno and Taniguchi, 1983; Dinter et al., 1983; Zinn et al., 1983). Thus cis-acting DNA sequences functioning as inducible enhancers have been identified within the 5'-flanking region of the human IFN-$\beta$ gene (Fujita et al., 1985, 1987; Goodbourn et al., 1985; Dinter and Hauser, 1987). The inducible enhancer region (i.e. -65 to -105 with respect to the CAP site) contains repetitive hexanucleotide units some of which indeed function in the induced-activation of transcription when multimerized (Fujita et al., 1987). We have identified in mammalian cells such as mouse L929 cells and human cells, a factor, IRF-1, which specifically binds to the IFN-$\beta$ regulatory sequences, as well as the functional, repeated hexanucleotide sequences; (AAGTGA)$_4$. We have found that IRF-1 plays an essential role in virus-induced activation of IFN-$\beta$ gene transcription by interacting with the identified cis-elements.

The present invention provides a recombinant DNA molecule is characterised by a structural gene having the Formula I below:

Formula I

```
          10        20        30        40        50
ATGCCCATCACTTGGATGCGCATGAGACCCTGGCTAGAGATGCAGATTAA

          60        70        80        90       100
TTCCAACCAAATCCCGGGGCTCATCTGGATTAATAAAGAGGAGATGATCT

         110       120       130       140       150
TGGAGATCCCATGGAAGCATGCTGCCAAGCATGGCTGGGACATCAACAAG

         160       170       180       190       200
GATGCCTGTTTGTTCCGGAGCTGGGCCATTCACACAGGCCGATACAAAGC

         210       220       230       240       250
AGGGGAAAAGGAGCCAGATCCCAAGACGTGGAAGGCCAACTTTCGCTGTG

         260       270       280       290       300
CCATGAACTCCCTGCCAGATATCGAGGAGGTGAAAGACCAGAGCAGGAAC

         310       320       340       340       350
AAGGGCAGCTCAGCTGTGCGAGTGTACCGGATGCTTCCACCTCTCACCAA

         360       370       380       390       400
GAACCAGAGAAAAGAAAGAAAGTCGAAGTCCAGCCGAGATGCTAAGAGCA

         410       420       430       440       450
AGGCCAAGAGGAAGTCATGTGGGGATTCCAGCCCTGATACCTTCTCTGAT

         460       470       480       490       500
GGACTCAGCAGCTCCACTCTGCCTGATGACCACAGCAGCTACACAGTTCC

         510       520       530       540       550
AGGCTACATGCAGGACTTGGAGGTGGAGCAGGCCCTGACTCCAGCACTGT

         560       570       580       590       600
CGCCATGTGCTGTCAGCAGCACTCTCCCCGACTGGCACATCCCAGTGGAA

         610       620       630       640       650
GTTGTGCCGGACAGCACCAGTGATCTGTACAACTTCCAGGTGTCACCCAT

         660       670       680       690       700
GCCCTCCATCTCTGAAGCTACAACAGATGAGGATGAGGAAGGGAAATTAC

         710       720       730       740       750
CTGAGGACATCATGAAGCTCTTGGAGCAGTCGGAGTGGCAGCCAACAAAC

         760       770       780       790       800
GTGGATGGGAAGGGGTACCTACTCAATGAACCTGGAGTCCAGCCCACCTC

         810       820       830       840       850
TGTCTATGGAGACTTTAGCTGTAAGGAGGAGCCAGAAATTGACAGCCCAG

         860       870       880       890       900
```

4

GGGGGGATATTGGGCTGAGTCTACAGCGTGTCTTCACAGATCTGAAGAAC

```
            910        920        930        940        950
ATGGATGCCACCTGGCTGGACAGCCTGCTGACCCCAGTCCGGTTGCCCTC

            960        970
CATCCAGGCCATTCCCTGTGCACCG
```

or having the Formula III below:

```
ATG CCA ATC ACT CGA ATG CGG ATG AGA CCC TGG CTA GAG ATG CAG ATT
AAT TCC AAC CAA ATC CCA GGG CTG ATC TGG ATC AAT AAA GAA GAG ATG
ATC TTC CAG ATT CCA TGG AAG CAC GCT GCT AAG CAC GGC TGG GAC ATC
AAC AAG GAT GCC TGT CTG TTC CGG AGC TGG GCC ATT CAC ACA GGC CGA
TAC AAA GCA GGA GAA AAA GAG CCA GAT CCC AAG ACA TGG AAG GCA AAC
TTC CGT TGT GCC ATG AAC TCC CTG CCA GAC ATC GAG GAA GTG AAG GAT
CAG AGT AGG AAC AAG GGC AGC TCT GCT GTG CGG GTG TAC CGG ATG CTG
CCA CCC CTC ACC AGG AAC CAG AGG AAA GAG AGA AAG TCC AAG TCC AGC
CGA GAC ACT AAG AGC AAA ACC AAG AGG AAG CTG TGT GGA GAT GTT AGC
CCG GAC ACT TTC TCT GAT GGA CTC AGC AGC TCT ACC CTA CCT GAT GAC
CAC AGC AGT TAC ACC ACT CAG GGC TAC CTG GGT CAG GAC TTG GAT ATG
GAA AGG GAC ATA ACT CCA GCA CTG TCA CCG TGT GTC GTC AGC AGC AGT
CTC TCT GAG TGG CAT ATG CAG ATG GAC ATT ATA CCA GAT AGC ACC ACT
GAT CTG TAT AAC CTA CAG GTG TCA CCC ATG CCT TCC ACC TCC GAA GCC
GCA ACA GAC GAG GAT GAG GAA GGG AAG ATA GCC GAA GAC CTT ATG AAG
CTC TTT GAA CAG TCT GAG TGG CAG CCG ACA CAC ATC GAT GGC AAG GGA
TAC TTG CTC AAT GAG CCA GGG ACC CAG CTC TCT TCT GTC TAT GGA GAC
TTC AGC TGC AAA GAG GAA CCA GAG ATT GAC AGC CCT CGA GGG GAC ATT
GGG ATA GGC ATA CAA CAT GTC TTC ACG GAG ATG AAG AAT ATG GAC TCC
ATC ATG TGG ATG GAC AGC CTG CTG GGC AAC TCT GTG AGG CTG CCG CCC
TCT ATT CAG GCC ATT CCT TGT GCA CCA TAG
```

or a fragment, or a degenerate variant of the molecule or fragment, which codes for a protein having the activity of an interferon regulatory factor 1 (IRF-1).

The present invention also comprehends a recombinant DNA sequence which hybridises to the antisense version of a molecule of the invention as defined above and which codes for a protein having IRF-1 activity.

The IRF-1 activity is to be understood the ability of the proteins to bind to the repeated oligomer sequence AAGTGA and the regulatory upstream elements of the human IFN-$\beta$-gene.

The DNA molecule may for example be characterized by a structural gene having the Formula I defined above and upstream and downstream flanking sequences contained within the following Formula II:

<u>Formula II</u>

CGAGCCCCGCCGAACCGAGGCCACCCGGAGCCGTGCCCAGTCCACGC

CGGCCGTGCCCGGCGGCCTTAAGAACCAGGCAACCACTGCCTTCTTCCCT

CTTCCACTCGGAGTCGCGCTTCGCGCGCCCTCACTGCAGCCCCTGCGTCG

CCGGGACCCTCGCGCGCGACCAGCCGAATCGCTCCTGCAGCAGAGCCAAC

```
          10        20        30        40        50
ATGCCCATCACTTGGATGCGCATGAGACCCTGGCTAGAGATGCAGATTAA

          60        70        80        90        100
TTCCAACCAAATCCCGGGGCTCATCTGGATTAATAAAGAGGAGATGATCT
          110       120       130       140       150
TGGAGATCCCATGGAAGCATGCTGCCAAGCATGGCTGGGACATCAACAAG

          160       170       180       190       200
GATGCCTGTTTGTTCCGGAGCTGGGCCATTCACACAGGCCGATACAAAGC

          210       220       230       240       250
AGGGGAAAAGGAGCCAGATCCCAAGACGTGGAAGGCCAACTTTCGCTGTG

          260       270       280       290       300
CCATGAACTCCCTGCCAGATATCGAGGAGGTGAAAGACCAGAGCAGGAAC

          310       320       340       340       350
AAGGGCAGCTCAGCTGTGCGAGTGTACCGGATGCTTCCACCTCTCACCAA
```

```
          360        370        380        390        400
GAACCAGAGAAAAGAAAGAAAGTCGAAGTCCAGCCGAGATGCTAAGAGCA

          410        420        430        440        450
AGGCCAAGAGGAAGTCATGTGGGGATTCCAGCCCTGATACCTTCTCTGAT

          460        470        480        490        500
GGACTCAGCAGCTCCACTCTGCCTGATGACCACAGCAGCTACACAGTTCC

          510        520        530        540        550
AGGCTACATGCAGGACTTGGAGGTGGAGCAGGCCCTGACTCCAGCACTGT

          560        570        580        590        600
CGCCATGTGCTGTCAGCAGCACTCTCCCCGACTGGCACATCCCAGTGGAA

          610        620        630        640        650
GTTGTGCCGGACAGCACCAGTGATCTGTACAACTTCCAGGTGTCACCCAT

          660        670        680        690        700
GCCCTCCATCTCTGAAGCTACAACAGATGAGGATGAGGAAGGGAAATTAC

          710        720        730        740        750
CTGAGGACATCATGAAGCTCTTGGAGCAGTCGGAGTGGCAGCCAACAAAC

          760        770        780        790        800
GTGGATGGGAAGGGGTACCTACTCAATGAACCTGGAGTCCAGCCCACCTC

          810        820        830        840        850
TGTCTATGGAGACTTTAGCTGTAAGGAGGAGCCAGAAATTGACAGCCCAG

          860        870        880        890        900
GGGGGGATATTGGGCTGAGTCTACAGCGTGTCTTCACAGATCTGAAGAAC

          910        920        930        940        950
ATGGATGCCACCTGGCTGGACAGCCTGCTGACCCCAGTCCGGTTGCCCTC

          960        970
CATCCAGGCCATTCCCTGTGCACCGTAGCAGGGCCCCTGGGCCCCTCTTA

TTCCTCTAGGCAAGCAGGACCTGGCATCATGGTGGATATGGTGCAGAGAA

GCTGGACTTCTGTGGGCCCCTCAACAGCCAAGTGTGACCCCACTGCCAAG

TGGGGATGGGCCTCCCTCCTTGGGTCATTGACCTCTCAGGGCCTGGCAGG

CCAGTGTCTGGGTTTTTCTTGTGGTGTAAAGCTGGCCCTGCCTCCTGGGA

AGATGAGGTTCTGAGACCAGTGTATCAGGTCAGGGACTTGGACAGGAGTC

AGTGTCTGGCTTTTTCCTCTGAGCCCAGCTGCCTGGAGAGGGTCTCGCTG

TCACTGGCTGGCTCCTAGGGGAACAGACCAGTGACCCCAGAAAAGCATAA

CACCAATCCCAGGGCTGGCTCTGCACTAAGAGAAAATTGCACTAAATGAA
```

TCTCGTTCCAAAGAACTACCCCTTTTCAGCTGAGCCCTGGGGACTGTTCC

AAAGCCAGTGAATGTGAAGGAAAGTGGGGTCCTTCGGGGCAATGCTCCCT

CAGCCTCAGAGGAGCTCTACCCTGCTCCCTGCTTTGGCTGAGGGGCTTGG

GAAAAAACTTGGCACTTTTTCGTGTGGATCTTGCCACATTTCTGATCAG

AGGTGTACACTAACATTTCCCCCGAGCTCTTGGCCTTTGCATTTATTTAT

ACAGTGCCTTGCTCGGGGCCCACCACCCCCTCAAGCCCCAGCAGCCCTCA

ACAGGCCCAGGGAGGGAAGTGTGAGCGCCTTGGTATGACTTAAAATTGGA

AATGTCATCTAACCATTAAGTCATGTGTGAACACATAAGGACGTGTGTAA

ATATGTACATTTGTCTTTTTATAAAAAGTAAAATTGTT

or a degenerate variant thereof.

The DNA molecule may, for example, be characterized by a structural gene having the Formula III defined above and upstream and downstream flanking sequences contained within the following formula IV:

## Formula IV

```
  1 GGACGTGCTTTCACAGTCTAAGCCGAACCGAACCGAACCGAACCGAACCGAACCGGGCC
 60 GAGTTGCGCCGAGGTCAGCCGAGGTGGCCAGAGGACCCCAGCATCTCGGGCATCTTTCG
119 CTTCGTGCGCGCATCGCGTACCTACACCGCAACTCCGTGCCTCGCTCTCCGGCACCCTC
178 TGCGAATCGCTCCTGCAGCAA   AGCCACC ATG CCA ATC ACT CGA ATG CGG
227 ATG AGA CCC TGG CTA GAG ATG CAG ATT AAT TCC AAC CAA ATC CCA
272 GGG CTG ATC TGG ATC AAT AAA GAA GAG ATG ATC TTC CAG ATT CCA
317 TGG AAG CAC GCT GCT AAG CAC GGC TGG GAC ATC AAC AAG GAT GCC
362 TGT CTG TTC CGG AGC TGG GCC ATT CAC ACA GGC CGA TAC AAA GCA
407 GGA GAA AAA GAG CCA GAT CCC AAG ACA TGG AAG GCA AAC TTC CGT
452 TGT GCC ATG AAC TCC CTG CCA GAC ATC GAG GAA GTG AAG GAT CAG
```

```
 497  AGT AGG AAC AAG GGC AGC TCT GCT GTG CCG GTG TAC CGG ATG CTG

 542  CCA CCC CTC ACC AGG AAC CAG AGG AAA GAG AGA AAG TCC AAG TCC

 587  AGC CGA GAC ACT AAG AGC AAA ACC AAG AGG AAG CTG TGT GGA GAT

 632  GTT AGC CCG GAC ACT TTC TCT GAT GGA CTC AGC AGC TCT ACC CTA

 677  CCT GAT GAC CAC AGC AGT TAC ACC ACT CAG GGC TAC CTG GGT CAG

 722  GAC TTG GAT ATG GAA AGG GAC ATA ACT CCA GCA CTG TCA CCG TGT

 767  GTC GTC AGC AGC AGT CTC TCT GAG TGG CAT ATG CAG ATG GAC ATT

 812  ATA CCA GAT AGC ACC ACT GAT CTG TAT AAC CTA CAG GTG TCA CCC

 857  ATG CCT TCC ACC TCC GAA GCC GCA ACA GAC GAG GAT GAG GAA GGG

 902  AAG ATA GCC GAA GAC CTT ATG AAG CTC TTT GAA CAG TCT GAG TGG

 947  CAG CCG ACA CAC ATC GAT GCC AAG GGA TAC TTG CTC AAT GAG CCA

 992  GGG ACC CAG CTC TCT TCT GTC TAT GGA GAC TTC AGC TGC AAA GAG

1037  GAA CCA GAG ATT GAC AGC CCT CGA GGG GAC ATT GGG ATA GGC ATA

1082  CAA CAT GTC TTC ACG GAG ATG AAG AAT ATG GAC TCC ATC ATG TGG

1127  ATG GAC AGC CTG CTG GGC AAC TCT GTG AGG CTG CCG CCC TCT ATT

1172  CAG GCC ATT CCT TGT GCA CCA TAG  TTTGGGTCTCTGACCCGTTCTTGCCC

1222  TCCTGAGTGAGTTAGGCCTTGGCATCATGGTGGCTGTGATACAAAAAAAGCTAGACTCC

1281  TGTGGGCCCCTTGACACATGGCAAAGCATAGTCCCACTGCAAACAGGGGACCATCCTCC

1340  TTGGGTCAGTGGGCTCTCAGGGCTTAGGAGGCAGAGTCTGAGTTTTCTTGTGAGGTGAA

1399  GCTGGCCCTGACTCCTAGGAAGATGGATTGGGGGGTCTGAGGTGTAAGGCAGAGGCCAT

1458  GGACAGGAGTCATCTTCTAGCTTTTTAAAAGCCTTGTTGCATAGAGAGGGTCTTATCGC

1517  TGGGCTGGCCCTGAGGGGAATAGACCAGCGCCCACAGAAGAGCATAGCACTGGCCCTAG

1576  AGCTGGCTCTGTACTAGGAGACAATTGCACTAAATGAGTCCTATTCCCAAAGAACTGCT

1635  GCCCTTCCCAACCGAGCCCTGGGATGGTTCCCAAGCCAGTGAAATGTGAAGGGAAAAAA

1694  AATGGGGTCCTGTGAAGGTTGGCTCCCTTAGCCTCAGAGGGAATCTGCCTCACTACCTG

1753  CTCCAGCTGTGGGGCTCAGGAAAAAAAAATGGCACTTTCTCTGTGGACTTTGCCACATT

1812  TCTGATCAGAGGTGTACACTAACATTTCTCCCCAGTCTAGGCCTTTGCATTTATTTATA

1871  TAGTGCCTTGCCTGGTGCCTGCTGTCTCCTCAGGCCTTGGCAGTCCTCAGCAGGCCCAG

1930  GGAAAAGGGGGGTTGTGAGCGCCTTGGCGTGACTCTTGACTATCTATTAGAAACGCCAC

1989  CTAACTGCTAAATGGTGTTTGGTCATGTGGTGGACCTGTGTAAATATGTATATTTGTCT

2048  TTTTATAAAAATTTAAGTTGTTTACAAAAAAAAAA 2082
```

or a degenerate variant thereof.

The DNA molecule may include a promoter region which is constitutive or inducable, for instance virus inducible e.g. by Newcastle Disease Virus or is inducable by mitogenic stimulation e.g. using Concanavalin A.

DNA sequences of the invention coding for proteins having IRF-I activity include DNA sequences from a eukaryotic source such as, not only human and mouse but also, e.g. chicken, frog and yeast, which hybridise with the DNA sequences of the foregoing human or murine IRF-I (Figs. I to IV) and which code for a protein having IRF-I activity.

We describe below the isolation of cDNA molecules from human cells and mouse cells coding for human and murine IRF-I and from yeast which hybridises to the cDNA of human IRF-I.

The recombinant DNA molecule may also contain a promoter and regulatory sequence contained within the following formula V:

## Formula V

```
                        PstI
                CTGCAGAAAGAGGGGGACGGTCTCGGCTTTCCAAGACAGGCAAGGGGG
                -299

                                GC Box 1                      GC Box 2
        CAGGGGAGTGGAGTGGAGCAAGGGGCGGGCCCGCGGTAGCCCCGGGGCGGTGGCGCGG
        -251


        GCCCGAGGGGGTGGGGAGCACAGCTGCCTTGTACTTCCCCTTCGCCGCTTAGCTCTAC
        -193

                                                CAAT Box
        AACAGCCTGATTTCCCCGAAATGATGAGGCCGAGTGGGCCAATGGGCGCGCAGGAGCG
        -135

                                                        Minor Cap site
                                                            ! ! !
        GCGCGGCGGGGGCGTGGCCGAGTCCGGGCCGGGGAATCCCGCTAAGTGTTTAGATTTC
        -77                                                         -21

                Major Cap site                          plRF-L
                    ▶▶▶                                    |
        TTCGCGGCGCCGCGGACTCGCCAGTGCGCACCACTCCTTCGTCGAGGTAGGACGTGCT
        -19             +1


        TTCACAGTCTAAGCCGAACCGAACCGAACCGAACCGAACCGAACCGGGCCGAGTTGCG
        +39


        CCGAGGTCAGCCGAGGTGGCCAGAGGACCCCAGCATCTCGGGCATCTTTCGCTTCGTG
        +97

        CGCGCATCGCGTACCTACACCGCAACTCCGTGCCTCGCTCTCCGGCACCCTCTGCGAA
        +155
                PstI
        TCGCTCCTGCAG
        +213      +225
```

or a degenerate variant thereof.

The recombinant DNA molecule may also be designed for expression of a pharmaceutically active protein such as e.g. a cytokine or a plasminogen activator and in this form will contain preferably a structural gene for a desired pharmaceutically active protein operably linked to a promoter region of the gene for the said protein including a binding site for the IRF-1 active molecule.

Thus a recombinant DNA molecule of the invention may comprise a DNA sequence as defined above and a structural gene for a desired pharmaceutically active protein under the control of the IRF-I active protein coded for by said DNA sequence. In such a recombinant DNA molecule the gene coding for the IRF-I active molecule is preferably under the control of a constitutive promoter or most preferably inducible promoter. Preferably the gene for the desired pharmaceutically active protein will include an IRF binding site containing repetitive AAGTGA sequences.

The present invention also comprehends a host cell e.g. a bacterial cell e.g. E. coli, or a yeast cell or a mammalian cell e.g. a CHO cell or a mouse cell e.g. L929, transformed by a recombinant DNA molecule as defined above. Ideally the host cell will be selected from a cell line which has no or substantially no level of endogenous IRF-I activity.

Alternatively for the production of a pharmaceutically active protein a host cell may be transformed by a first DNA molecule containing a sequence coding for a protein having the activity of an IRF-I, and by a second separate DNA molecule containing a gene coding for a desired, pharmaceutically active protein under the control of the IRF-I active protein coded for by the first DNA molecule. Preferably the first DNA molecule coding for the IRF-I active molecule includes a constitutive promoter or most preferably inducible promoter sequence operably linked to the gene coding for the IRF-I active compound. Also, preferably the second DNA molecule includes a binding site for the IRF-I active protein containing repetitive AAGTGA sequences.

The IRF-I active protein or the pharmaceutically active protein can be produced by cultivation of the transformed cell and isolation of the produced protein in conventional manner.

Suitably the host cells are induced by treatment in a manner appropriate to the promoter which is operably linked to the gene coding for the IRF-1, as discussed below.

The invention also comprehends a protein having the activity of an IRF-1 obtained by the cultivation of a host transformed with a DNA molecule as defined above.

A preferred protein having the activity of an IRF-I has the formula VI:

<u>Formula VI</u>

```
Met Pro Ile Thr Arg Met Arg Met Arg Pro Trp Leu Glu Met Gln Ile
Asn Ser Asn Gln Ile Pro Gly Leu Ile Trp Ile Asn Lys Glu Glu Met
Ile Phe Gln Ile Pro Trp Lys His Ala Ala Lys His Gly Trp Asp Ile
Asn Lys Asp Ala Cys Leu Phe Arg Ser Trp Ala Ile His Thr Gly Arg
Tyr Lys Ala Gly Glu Lys Glu Pro Asp Pro Lys Thr Trp Lys Ala Asn
Phe Arg Cys Ala Met Asn Ser Leu Pro Asp Ale Glu Glu Val Lys Asp
Gln Ser Arg Asn Lys Gly Ser Ser Ala Val Arg Val Tyr Arg Met Leu
Pro Pro Leu Thr Arg Asn Gln Arg Lys Glu Arg Lys Ser Lys Ser Ser
Arg Asp Thr Lys Ser Lys Thr Lys Arg Lys Leu Cys Gly Asp Val Ser
Pro Asp Thr Phe Ser Asp Gly Leu Ser Ser Ser Thr Leu Pro Asp Asp
His Ser Ser Tyr Thr Thr Gln Gly Tyr Leu Gly Gln Asp Leu Asp Met
Glu Arg Asp Ile Thr Pro Ala Leu Ser Pro Cys Val Val Ser Ser Ser
Leu Ser Glu Trp His Met Gln Met Asp Ile Ile Pro Asp Ser Thr Thr
Asp Leu Tyr Asn Leu Gln Val Ser Pro Met Pro Ser Thr Ser Glu Ala
Ala Thr Asp Glu Asp Glu Glu Gly Lys Ile Ala Glu Asp Leu Met Lys
Leu Phe Glu Gln Ser Glu Trp Gln Pro Thr His Ile Asp Gly Lys Gly
Tyr Leu Leu Asn Glu Pro Gly Thr Gln Leu Ser Ser Val Tyr Gly Asp
Phe Ser Cys Lys Glu Glu Pro Glu Ile Asp Ser Pro Arg Gly Asp Ile
Gly Ile Gly Ile Gln His Val Phe Thr Glu Met Lys Asn Met Asp Ser
Ile Met Trp Met Asp Ser Leu Leu Gly Asn Ser Val Arg Leu Pro Pro
Ser Ile Gln Ala Ile Pro Cys Ala Pro
```

Another preferred protein having the activity of an IRF-I has the formula VII:

Formula VII

```
                              MetProIleThrTrpMetArgMetArgProTrpLeuGluMet
GlnIleAsnSerAsnGlnIleProGlyLeuIleTrpIleAsnLysGluGluMetIleLeu
GluIleProTrpLysHisAlaAlaLysHisGlyTrpAspIleAsnLysAspAlaCysLeu
PheArgSerTrpAlaIleHisThrGlyArgTyrLysAlaGlyGluLysGluProAspPro
LysThrTrpLysAlaAsnPheArgCysAlaMetAsnSerLeuProAspIleGluGluVal
LysAspGlnSerArgAsnLysGlySerSerAlaValArgValTryArgMetLeuProPro
LeuThrLysAsnGlnArglysGluArgLysSerLysSerSerArgAspAlaLysSerLys
AlaLysArgLysSerCysGlyAspSerSerProAspThrPheSerAspGlyLeuSerSer
SerThrLeuProAspAspHisSerSerTyrThrValProGlyTyrMetGlnAspLeuGlu
ValGluGlnAlaLeuThrProAlaLeuSerProCysAlaValSerSerThrLeuProAsp
TrpHisIleProValGluValValProAspSerThrSerAspLeuTyrAsnPheGlnVal
SerProMetProSerIleSerGluAlaThrThrAspGluAspGluGluGlyLysLeuPro
GluAspIleMetLysLeuLeuGluGlnSerGluTrpGlnProThrAsnValAspGlyLys
GlyTryLeuLeuAsnGluProGlyValGlnProThrSerValTyrGlyAspPheSerCys
LysGluGluProGluIleAspSerProGlyGlyAspIleGlyLeuSerLeuGlnArgVal
PheThrAspLeuLysAsnMetAspAlaThrTrpLeuAspSerLeuLeuThrProValArg
LeuProSerIleGlnAlaIleProCysAlaPro
```

We describe below the molecular cloning and characterization of murine and human cDNA encoding DNA binding proteins having the IRF-1 activity.

A remarkable sequence conservation will be seen between the murine and human IRF-1 molecules, as revealed from the analysis of the cloned cDNAs. Furthermore, expression of the gene encoding IRF-1 is shown to be induced by Newcastle Disease Virus (NDV) and Concanavalin A (ConA) in mouse L929 cells and splenic lymphocytes, respectively.

1. Cloning and expression of IRF-1 DNA in E. coli

A. Poly(A)[+] RNA was isolated from uninduced mouse L929 cells and used to synthesis cDNA (following the procedure of Aruffo and Seed, 1987). The resulting cDNA was then cloned into an EcoRI-cleaved λgt11 vector and a cDNA library constructed according to the standard procedure of Huynh et al., 1985, using E. coli Y1090 as the host strain.

The resulting λgt11 library was then screened using the multimerized (4 times) AAGTGA sequence (hereinafter referred to as the C1 oligomer; Fujita et al., 1987) as the probe.

In this screening procedure E. coli Y1090, infected by the recombinant λgt11 phages was plated onto 10x13 cm square plates. The plates were then incubated at 12°C for 4 to 5 hours when about 20,000 plaques/plate were visible.

Membrane filters for screening were either nylon (Nytran; Schleicher & Schnell) or nitrocellulose (Schleicher & Schnell) membranes. The filters were immersed in 10 mM IPTG (for the induction of lacZ gene expression in the appropriate phage plaques) and air dried, then overlayed on the plates and incubated at 37°C for 2,5 hours. Plaques will only produce a protein encoded by cDNA if the cDNA is in-frame with the lacZ gene.

The filters were then removed and chilled at 4°C for 20 minutes and, without drying, were subjected to screening.

The membrane filters were then prepared for assay as follows:
Nuclear extract was prepared from mouse L929 cells and it was spotted (in an amount corresponding to

about 10 $\mu$g protein) onto the membranes.

Prior to effecting DNA binding a Binding Buffer consisting of 10 mM Hepes, pH 7,5, 5,0 mM NaCl, 1 mM DTT, 1 mM EDTA, 5% glycerol was used. 5% of non-fat powder milk (Yukijurishi Inc.) was added to the buffer and the filters were incubated in the mixture for 1 hour at 4°C and then rinsed for 1 minute in the same buffer but containing no powder milk.

The filters were then incubated in binding buffer (1 ml) containing 350 $\mu$g/ml of salmon sperm DNA of average length of approximately 300 bp and $^{32}$P-labelled probe C1 oligomer DNA ($10^6$ cpm/ml; specific activity 2,000 cpm/f mole) (see Fujita et al., 1987). The probe DNA was end labeled at the 5'-termini [$\gamma$-$^{32}$P]ATP using T4 kinase.

After the binding, the filters were washed at room temperature with the Binding Buffer for 1 to 2 hours (10 ml filter) changing the Binding buffer several times during this operation. The filters were then air-dried and subjected to autoradiography. In this assay the nylon membranes, but not the nitrocellulose membranes, gave a positive signal which was specifically inhibited by including excess unlabeled C1 oligomer.

About 1,4x$10^6$ recombinants were screened in this way. Among the 32 positive phage clones identified in the first screening, one clone (designated $\lambda$L28-8) was found to bind repeatedly with the probe DNA in subsequent rounds of screening.

## 2. Production and Purification of protein in transfected E. coli

Lysogenic bacterial clones were prepared by transfecting E.coli Y1089 with $\lambda$L28-8. Overnight lysogens harboring $\lambda$L28-8 were then seeded at 1% in 400 ml L-Broth. The bacteria were grown at 31°C until the OD$_{600}$ became 1. The temperature was then shifted to 42°C for 20 min. IPTG was then added to 10 mM and, after incubating at 38°C for a further 20 min. the cultures were rapidly pelleted and suspended in 10 ml of Lysis Buffer which consists of 20 mM Hepes pH 7.9, 0.2 mM EDTA, 0.5 mM spermidine, 0.15 mM spermine, 0.1 mM DTT, 10 % glycerol, 0.5 mM phenylmethyonylsulphonylfluoride (PMSF), 1 $\mu$g/ml pepstatin A, 1 $\mu$g/ml leupeptine, 500 $\mu$M L-I-tosylamide-2-phenylethylchloromethylbenzamidine, 10 mM sodium molybdate, 2 mM sodium pyrophosphate and 2 mM sodium orthovanadate. Cell suspensions were subjected to three rapid freeze-thaw cycles and subsequently centrifuged at 30,000 rpm for 1 hr. at 4°C using a Beckman 50 Ti rotor. The supernatant was used either directly for a gel retardation assay (see below) or was further purified.

Further purification was carried out as follows:

Approximately 4 ml of the supernatant was applied on poly(di-C): poly(di-C)-column with a bed volume of 2 ml, and equilibrated with Lysis Buffer. The flow-through material (4 ml) was then applied on a DE 52 (Whatman) column having a bed volume of 2 ml equilibrated with Buffer Z (25 mM Hepes, pH 7,8, 12,5 mM MgCl$_2$, 1 mM DTT, 20% glycerol, 0,1% NP-40, 0,5 mM PMSF). The DNA binding activity was eluted by Buffer Z containing 0,1 m KCl. The eluate (approximately 4 ml) was further concentrated using centricon-10 (Amicon). The final protein concentration was 28 mg/ml.

## 3. Characterisation of the Protein Product of the Clone $\lambda$L28-8:

### (1) Gel retardation assay

Lysogenic bacterial clones harbouring $\lambda$L28-8 were prepared by transfecting E. coli Y1089 and were induced to express the cloned cDNA at high levels using the procedure of Huynh et al., 1985. Lysogenic clones prepared and treated in the same manner with the phage lacking the cDNA insert (designated $\lambda$6) were used as a control.

Extracts each containing 3 $\mu$g protein were prepared from the induced cultures of the Lysogen (four preparations designated $\lambda$L28-8a, $\lambda$L28-8b and $\lambda$6a and $\lambda$6b).

Nuclear extract (3 $\mu$g protein) was also prepared from mouse L929 cells.

The extracts were incubated with 1 f.mole labeled C1 oligomer probe as described above having a specific activity of 8,000 cpm/f.mole.

Each extract was also subject to competitive assay in which a competitor DNA was added at various concentrations to the incubation.

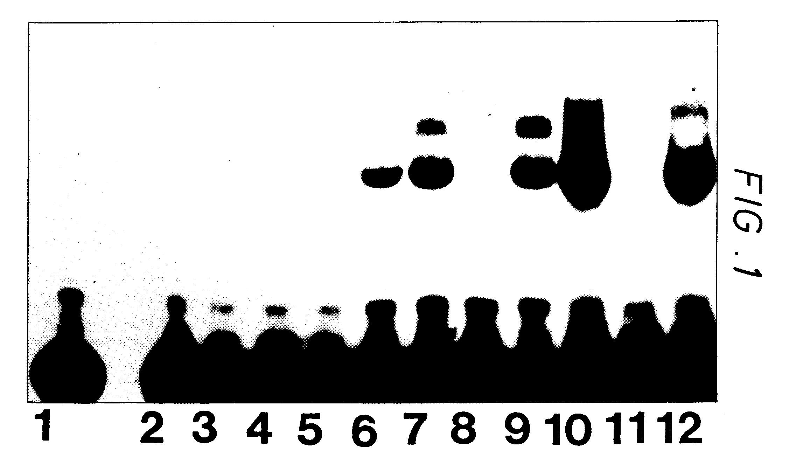

The results of the assay are shown in Fig. 1 in which the various lanes correspond to the following:

| Lane | Extract |
|---|---|
| 1 | none |
| 2 | λ6a |
| 3 | λ6b |
| 4 | λ6b with 1,000 fold molar excess of unlabeled C1 oligomer |

| Lane | Extract |
|---|---|
| 5 | λ6b with 1,000 fold molar excess of unlabeled C5A oligomer* |
| 6 | λL28-8a |
| 7 | λL28-8b |
| 8 | λL28-8b with 1,000 fold molar excess of unlabeled C1 oligomer |
| 9 | λL28-8b with 1,000 fold molar excess of unlabeled C5A oligomer* |
| 10 | L929 cell |
| 11 | L929 cell with 1,000 fold molar excess of unlabeled C1 oligomer |
| 12 | L929 cell with 1,000 fold molar excess of unlabeled C5A oligomer* |

\* The C5A oligomer is described in Fujita et al., 1985, and is a 6 times repeated GAAA sequence.

From Fig. 1 it will be apparent that bound probes are detectable in lanes 6, 7, 9, 10 and 12.

As shown in Fig. 1 protein extracts from the λL28-8 lysogens gave rise to shifted bands (lanes 6 and 7), the appearence of which was inhibited by excess unlabeled C1 oligomer DNA but not by the same amount of the C5A oligomer (lanes 8 and 9).

In contrast to λL28-8-derived proteins those prepared from the induced λ6-derived lysogens failed to give such shifted bands (lanes 2-5). The shifted bands can be seen to be closely similar to those of the natural IRF-1 from mouse L929 cells (lanes 10 and 12). Such differences as exist in the two sets of shifted bands is considered to be a consequence of the different amounts of protein bound to the probe DNA.

In addition, it is found that the shifted bands were detectable only with the proteins from IPTG-induced Y1089 cells transfected with λL28-8.

(ii) DNAase Footprinting analysis

Footprinting analysis was carried out to test the binding properties of the protein encoded by λL28-8 cDNA to a DNA encoding the IFN-$\beta$ gene upstream region.

Protein encoded by λL28-8 cDNA was extracted from the induced lysogen and partially purified by column chromatography as described above and tested for its binding properties to a DNA containing the IFN-$\beta$ gene upstream region.

Probe DNAs were prepared as SalI-HindIII fragments isolated from p-125cat (containing the wild type IFN-$\beta$ gene) and p-125DPcat (containing a mutant IFN-$\beta$ gene). The plasmid p-125cat was constructed as p-105cat (Fujita et al., 1987), except the BamHI (-125)-TagI (+19) fragment from pSE-125 (Fujita et al., Cell, Vol. 41, pp 489-496, 1985) was used. Plasmid p-125DPcat, carrying point mutations within the IFN-$\beta$ regulatory elements were obtained by synthetic oligo nucleotide directed mutagenesis on p-125cat as described in Hatakeyama et al., Proc. Natl. Acad. Sci. USA, Vol. 83, pp 9650-9654, 1986). Both DNAs were labeled at the HindIII site by ($\gamma$-$^{32}$P]ATP using T4 kinase.

4 f.moles of the probe DNA (specific activity 3.000 cpm/f.mole) was incubated in the 20 $\mu$l reaction mixture containing 25 mM Tris-HCl, pH 7,9, 6,25 mM MgCl$_2$, 50 mM KCl, 1 mM EDTA, 0,5 mM DTT, 10% glycerol, 2% polyvinylalcohol in the presence or absence of 280 $\mu$g of the purified protein.

In the assay 5x10$^{-4}$ unit of DNAase I (Worthington) was added and incubated for 1 min. at 25°C.

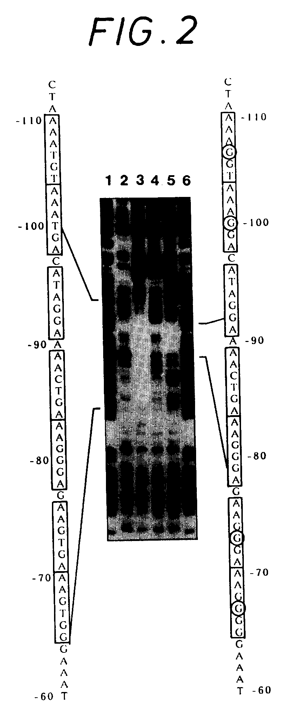

Fig. 2 shows autoradiograms of the DNA fragments obtained from samples obtained by carrying out the following procedures. On the left hand side of Fig. 2 is part of the DNA sequence of the wild type IFN-$\beta$ probe, and on the right hand side of Fig. 2 is part of the DNA sequence of the mutant IFN-$\beta$ probe.

1. The wild type IFN-$\beta$ probe was cleaved by A+G reactions (see Methods in Enzymology, Vol. 65, pp 499-560) - result shown in lane 1.

2. The wild type IFN-$\beta$ probe was partially digested by DNAase I without protection - result shown in lane 2.

3. The wild type IFN-$\beta$ probe was reacted with the protein and then digested with DNAase I - result shown in lane 3.

4. The wild type IFN-$\beta$ probe was reacted with the protein in the presence of 1 000 fold molar excess of unlabeled C1 oligomer and then digested with DNAase I - result shown in lane 4.

5. The mutant IFN-$\beta$ probe was reacted with the protein and then digested by DNAase I - result shown in lane 5.

6. The mutant IFN-$\beta$ probe was cleaved by A+G reactions - result shown in lane 6.

In Fig. 2 the protected regions as revealed in lanes 3 and 5 are indicated respectively on the sequences depicted on the left and right sides of the autoradiogram. The hexamer motifs are framed.

From the results in Figure 2 it will be seen that the protected region corresponds to nucleotides -100 to -64, this being the region found to be protected by IRF-1 obtained from L929 cells. The protection was abrogated by the use of excess unlabeled C1 oligomer (lane 4). It was also found, using lower protein concentrations, that preferential protection occurred in the region containing the AAGTGA motif (-80 to -70).

These results indicate that the protein has a higher affinity to the region containing the AAGTGA motif and a lower affinity to the surrounding region.

The mutant IFN-$\beta$ gene segment carries T- G mutations at positions -106, -100, -73 and -67. In comparing lanes 5 and 2, under the same assay conditions the protection afforded by the recombinant proteins appears restricted to the unmutated region (lane 2). This observation is in conformity with the observation that the introduction of these mutations results in a dramatic reduction (20 fold) of the inducibility of transcription by NDV in L929 cells and that in vitro binding of IRF-1 to the mutant IFN-$\beta$ gene was notable only in the unmutated region.

Further, the use of the C1 oligomer reveals that the protein specifically protects the region containing the oligomer sequences. This protection corresponded identically to that afforded by native IRF-1 derived from L929 cells.

3. DNA Competition Assay

This assay was carried out to examine the affinity of the recombinant protein to various DNA sequences including some of the known transcriptional regulatory DNA sequences. The procedure was as follows:

The HindIII-SalI fragment of IFN-$\beta$ probe was isolated from p-125 cat (see Fijita et al., 1987); this fragment contains the human IFN-$\beta$ gene sequence from +19 to -125. The DNA was labeled at the 3′ termini by filling in both ends with [$\alpha$-$^{32}$P] dCTP using Klenow fragment.

The specific activity of the probe DNA was 8 000 cpm/ f. mole.

The gel retardation assays were carried out under the conditions described above.

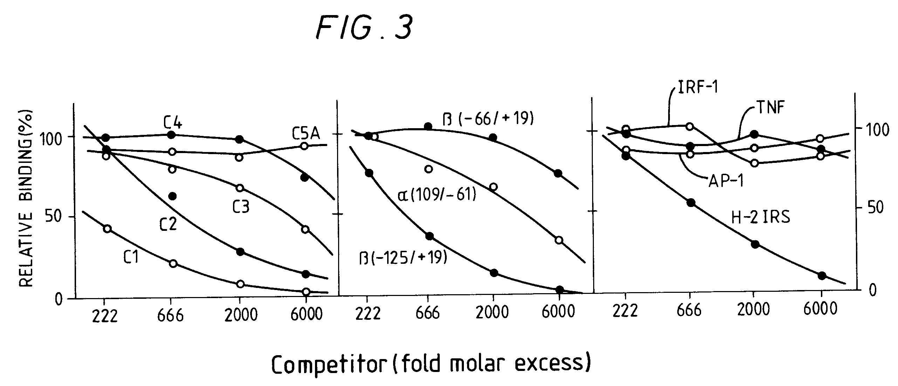

In the competition assay runs the DNA was reacted with the protein in the presence of various concentrations of competitor DNAs in the binding mixture as indicated in Figure 3.

The formation level of the complex was quantitated by densitometric analysis of the autoradiogram. Complex formation in the absence of competitor DNA was taken to be 100 %.

The structure of the competitor DNAs were as follows:

16

a) AP-1: a synthetic DNA having the following sequence

$$5\text{'CTAGA(TGACTCA)}_6\text{G 3'}$$
$$3\text{'T(ACTGAGT)}_6\text{CCTAG 5'} \qquad ;$$

b) TNF: 37 bp of synthetic DNA that encompasses from +1162 to +2116 (see Nedwin et al., 1985) of the TNF-$\alpha$ first intron;

c) murine H2-D$^d$: 37 bp of synthetic DNA that encompasses the IRS element (-159 to -123) as described by Korber et al., 1988;

d) human IFN-$\alpha$: 46 bp synthetic DNA corresponding to virus Response Element, see Ryals et al., 1985.

e) Hexamer sequences C1, C2, C3, C4 and C5A.

The sequences C1 and C5A are as described above. Sequences C2, C3 and C4 are as published in Cell, 49, 352-367 (1987); they represent the sequences

| | |
|---|---|
| AAATGA | C2 |
| AAGGGA | C3 and |
| AAAGGA | C4 respectively |

f) the IFN-$\beta$ gene sequence from +19 to -66.

In Fig. 3 the left hand panel shows the results obtained when the hexamer repeats were used as competitors. The middle panel gives the results when human IFN gene segments were used as competitors, and the right hand panel gives the corresponding results, using various DNA segments as indicated within the panel.

From the results shown in Fig. 3 it will be seen that the appearance of the shifted band was competed out by the hexamer sequences in order of efficiency C1- C2- C3- C4, but was not competed out significantly by C5A.

It can also be observed that the synthetic DNA segments encompassing the regulatory elements of either human IFN-$\alpha$1 or murine H-2D$^d$ genes gave rise to a competitive activity. This is particularly interesting as the DNA segment of the H-2D$^d$ gene contains the so-called IFN-response sequence (IRS) that functions as an enhancer when the cells respond to IFN (Sugita et al., 1987; Israel et al., 1986; Korber et al., 1988).

In fact, sequence motifs similar or identical to these found on the IFN-$\beta$ gene are found in many of the promoter sequences of the IFN-inducible genes where nuclear factors appear to bind specifically (Korber et al., 1988; Lery et al., 1988).

The results given in Fig. 3 are closely similar to those obtained when the assay is repeated under similar conditions using natural protein produced from L929 cells.

## Structure of the cDNA encoding murine IRF-I

The DNA sequence of the cloned DNAs was determined either by a dideoxy method (SEQUENASE; United States Biochemical, Inc.) or by the standard Maxam-Gilbert method (Maxam and Gilbert, 1980).

The λL28-8 insert in E.Coli Y1089 was isolated as follows: the phage DNA was prepared by standard procedure and the DNA was digested by EcoRI then the cDNA cleaved out of the phage DNA was isolated and sequenced by the dideoxy method (Sequenase: United States Biochemical Inc.). The cDNA insert in λL28-8 was found to be 1.8 kb long. The nucleotide sequence analysis revealed a large open reading frame linked in phase with the $\beta$-galactoside gene.

To screen clones containing larger cDNA inserts, double stranded cDNA was synthesised with L929 cell derived poly (A)$^+$RNA and cloned into vector CDM8 according to the published procedure of Aruffo and Seed (Prod. Natl. Acad. Sci., USA, Vol 84, pp 8573, 1987) and Seed (Nature, Vol. 329, pp. 840-842, 1987).

The recombinant plasmids were introduced into E.coli strain MC1061/p3 (according to the procedure of Aruffo and Seed; as above) and the clones of cDNA were screened using the λL28-8-derived ($^{32}$p-labeled) cDNA probe under low stringent conditions for DNA-DNA hybridisation (Kashima et al, Nature, Vol. 313, pp 402-404, 1985), and a clone pIRF-L was selected for further study.

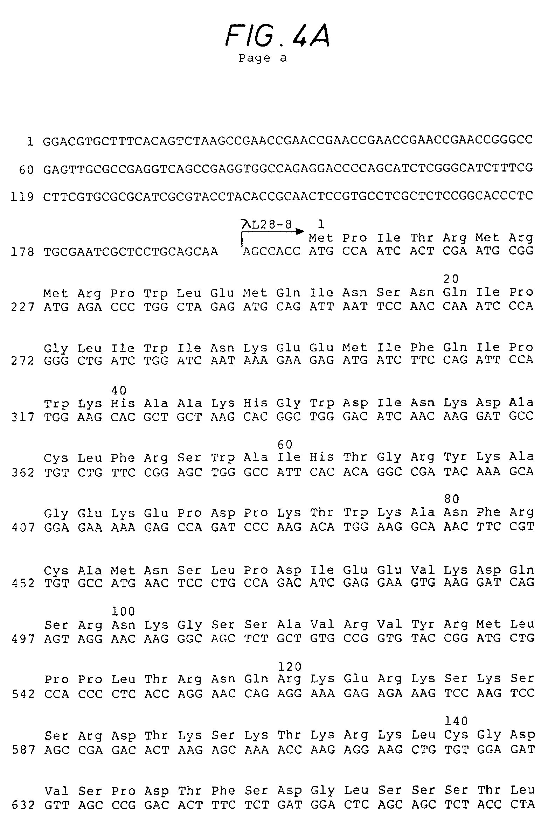

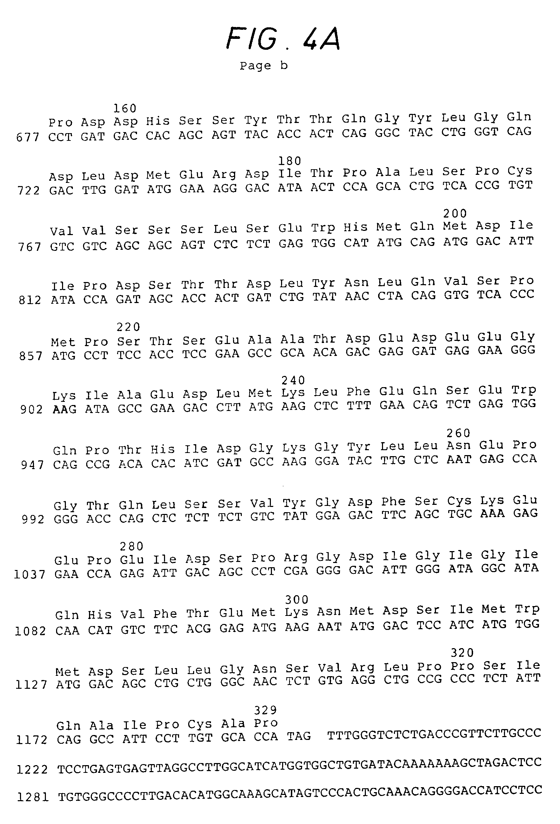

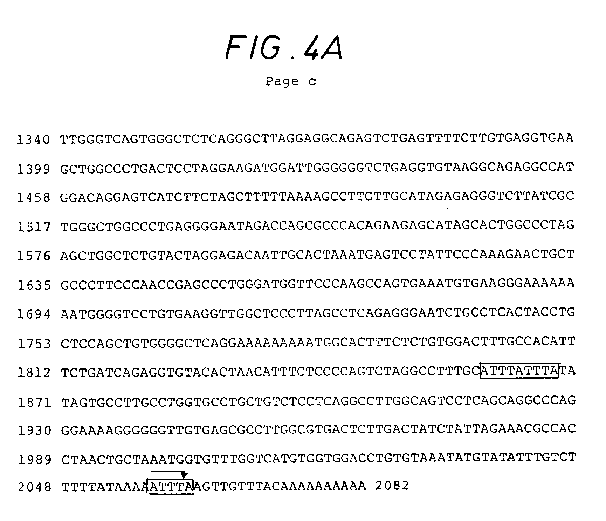

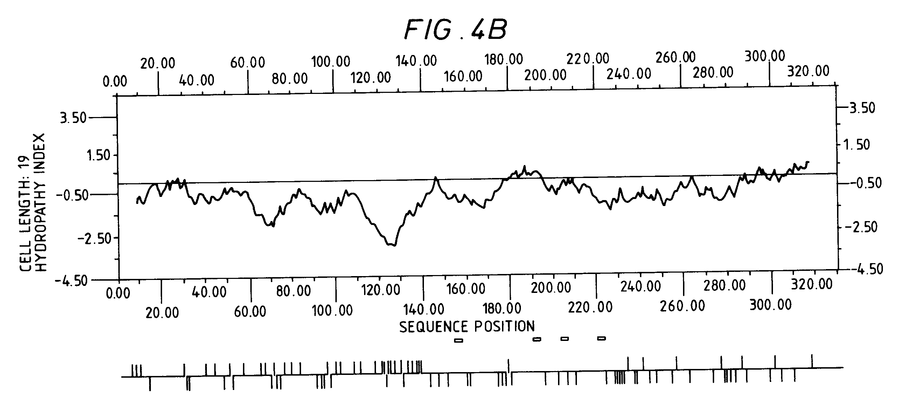

The desired cDNA insert of pIRF-L was obtained by digestion with HindIII and XbaI and sequenced by the methods described above. The sequence is shown in Formula IV and in Figure 4.

The cDNA sequence from λL28-8 was found to contain an identical sequence in the overlapping region except that one A residue was missing between nucleotides 1773 and 1781.

The 5′ and 3′ termini of the λL28-8 derived cDNA are marked by arrows in Fig. 4. The ATTTATTTA and ATTTA sequences which possibly confer the mRNA instability are framed.

As will be apparent from Fig. 4A the cDNA of the murine cDNA derived from pIRF-L was 198 bp and 20 bp longer than that of the λL28-8 cDNA in the 5′and 3′ regions, respectively.

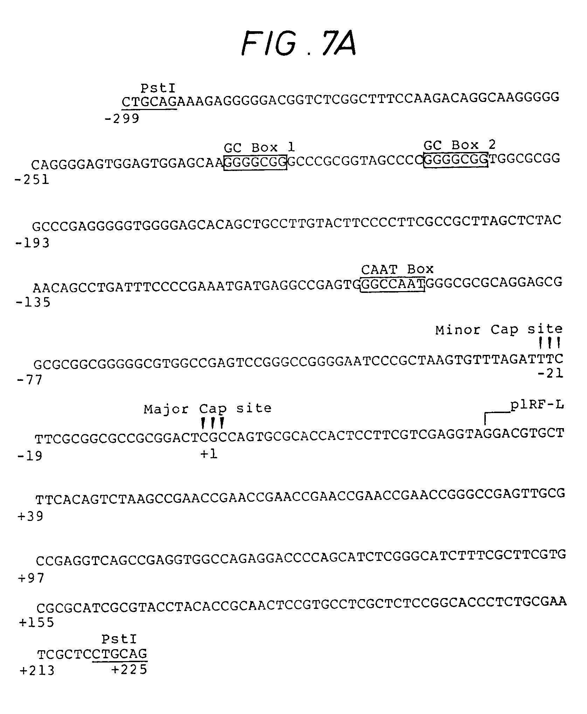

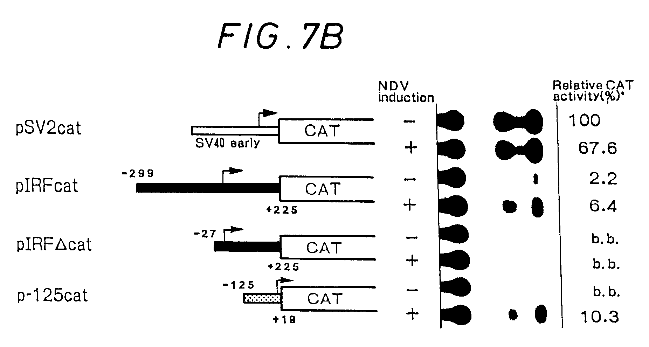

Analysis of the genomic DNA sequence containing the promoter region of this gene reveals that the cDNA of pIRF-L is missing about 30 bp from the major CAP site, see below and Formula V and Figure 7.

The immediate upstream sequence of the first ATG codon, GGACC$\underline{ATG}$C, fits well with Kozak's consensus sequence GCC$^A_G$ CC$\underline{ATG}$G, for the translation initation site.

An in-frame ATG sequence was not found in the upstream sequence from the above mentioned ATG sequence confirming that it is indeed the initiation codon for the IRF-I mRNA.

As mentioned above, no difference in nucleotide sequence was detected between the cDNAs of λL28-8 and pIRF-L within the overlapping regions, except one nucleotide in the 3′-non-coding region.

The murine IRF-I was thus found to consist of 329 amino acids with a calculated M.W. of 37.3 KD. Canonical N-glycosylation sites do not appear within the sequence.

No significant homology to other known proteins was detected by searching in Protein Sequence Database (Natl. Biomed. Res. Found., Washington, D.C.) and more recently published sequences.

Hydropathy plot analysis according to Kyte and Doolittle, 1982, indicates that the protein as a whole is highly hydrophilic (Figure 4B).

Inspection of the deduced primary sequence of the murine IRF-I reveals the following features:

The amino terminal half, extending to amino acid (a.a.) 140 is rich in lysin (Lys) and arginine (Arg). In fact 31 out of 39 of the total Lys and Arg residues are located in this region. In the lower panel of Fig. 4B is represented a diagrammatic summary of the location of the basic amino acids (Arg, Lys) (upward columns) and acidic amino acids (Asp, Glu) (downward columns).

As shown in Fig. 4B, this region shows strong hydrophilicity and is considered to be the region primarily responsible for the binding of IRF-I to the specific DNA sequences.

In this connection, characteristic motifs for many DNA binding proteins such as Zinc fingers and helix-turn-helix motifs (Pabo and Sauer, 1984, Evans and Hollenberg, 1988) were not detectable in the IRF-I protein.

In contrast the rest of the molecule (i.e. the carboxyl terminal half) shows a relative abundance of aspartic acid (Asp), glutamic acid (Glu), Serine (Ser) and Threonine (Thr). Of 189 amino acids (from a.a. 140 to 329), 33 (17 %) represent acidic amino acids and 36 (19 %) represent Ser and Thr. Notably a cluster of 5 consecutive acidic amino acids is found in a.a. 227 to 231. With regard to Ser and Thr, many appear to form clusters (region at a.a. 153-156, 190-192, 206-208, 220-222; referred to as the S-T regions herein). The S-T regions are depicted by small open rectangles in the lower panel of Fig. 4B.

## Structure of the cDNA encoding human IRF-I

Following a procedure similar to that described above for the murine IRF-I, human IRF-I cDNA was cloned and sequenced.

A human cDNA library was prepared by synthesising cDNA using poly(A)$^+$RNA from a human T cell line Jurkat. The double stranded cDNA synthesis and subsequent cloning into plasmid vector CDM8 was carried out according to the procedure of Aruffo and Seed (Proc. Natl., Acad. Sci., USA, Vol. 84, pp 8573-8577, 1987) and Seed (Nature, Vo. 329, pp 840-842, 1987).

The recombinant plasmids were introduced into E.coli strain, MC1061/p3 using the procedure of Aruffo and Seed as mentioned above.

Clones of cDNA that cross-hybridize with mouse IRF-I cDNA were screened using λL28-8 cDNA ($^{32}$p-labeled) as the probe under low stringent conditions for DNA-DNA hybridisation. The conditions employed were exactly as described by Kashima et al. (Nature, Vol. 313, pp 402-404, 1985).

From the positive clones clone pHIRF31 containing the longest cDNA insert was selected.

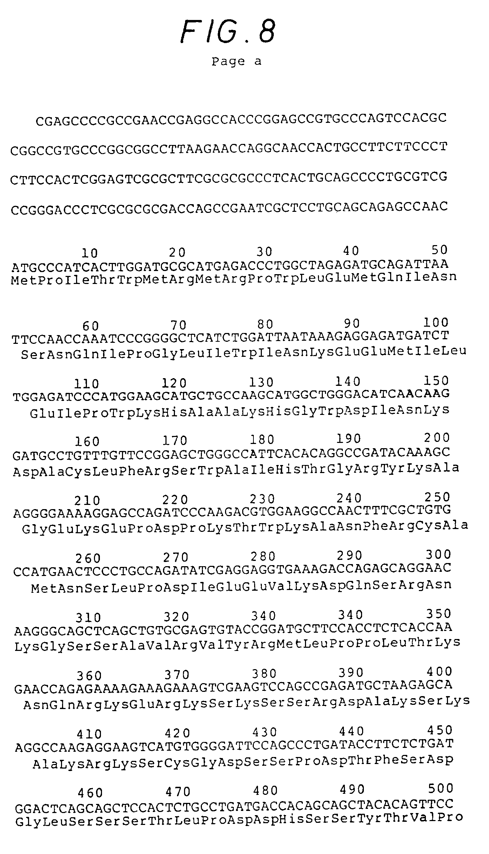

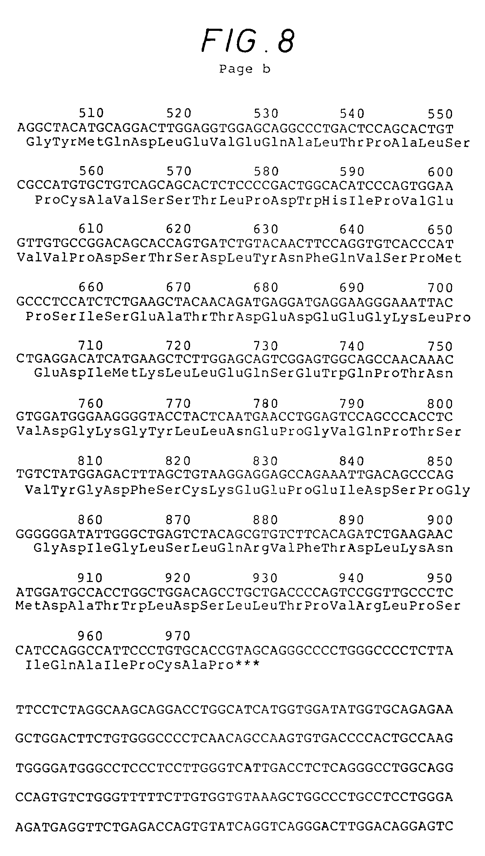

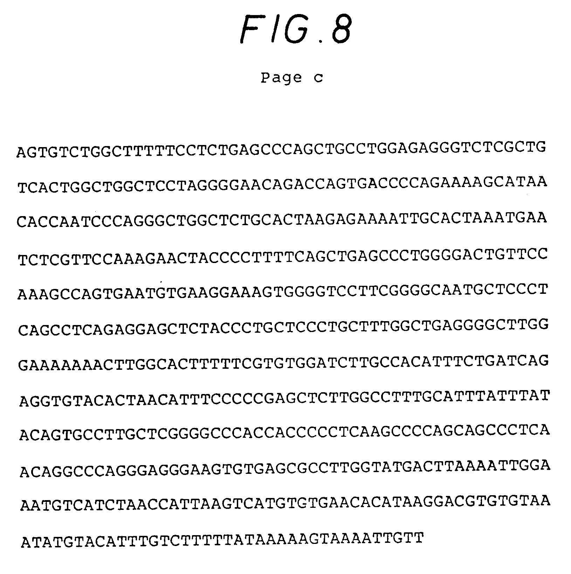

The desired cDNA sequence of clone pHIRF31 was isolated by digestion of the plasmid DNA by XhoI and after isolation subjected to sequencing by the methods described above. The structure of the human IRF-I gene is shown in Formula II and in Fig. 8.

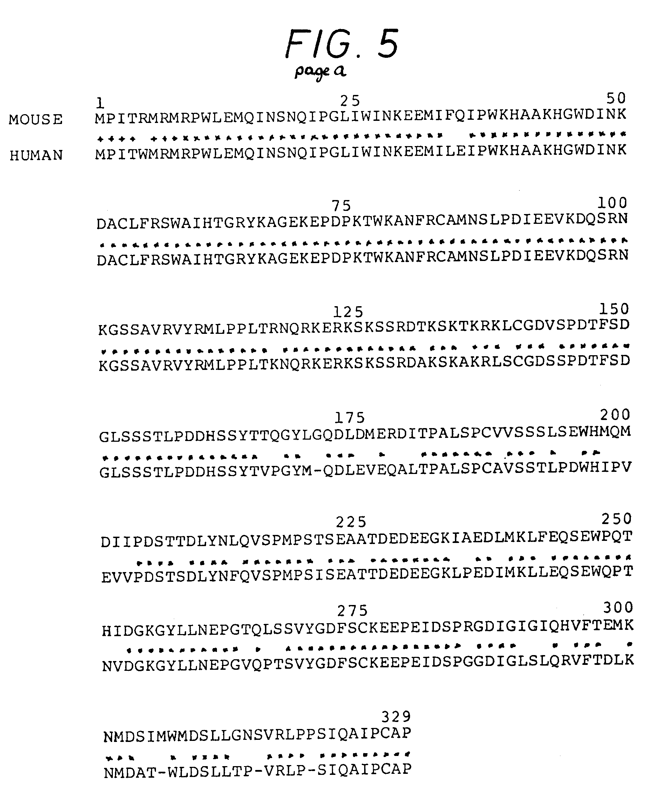

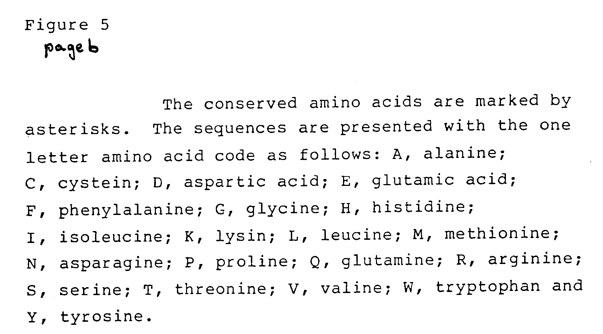

The sequences for the deduced murine and human IRF-I are shown juxtaposed for comparison in Fig. 5.

Analysis of the human DNA revealed that this IRF-I is shorter than the murine IRF-I by four amino acids.

Strong conservation of the amino acid sequences can be seen between the two IRF-I molecules. In particular, 133 out of 140 amino acids (95 %) of the amino terminal halves can be seen to be identical.

Taken together, the above observation indicate that IRF-I is a new class of DNA binding protein.

It should also be noted that the sequence ATTTATTTA and ATTTA, found in many cytokine and proto-oncogene mRNAs, are present within the 3′ non-translated region of the murine IRF-I cDNA, and likewise the sequence ATTTA is found within the corresponding region of the human IRF-I cDNA. These sequences are believed to play a role in the post-transcriptional regulation of gene expression by confering instability to the mRNA (Shaw and Kamen, 1986; Caput et al., 1986).

Plasmid pIRF-L was transfected into E.coli MC1061/p3 which was deposited as E.coli MC106/p3 (pIRF-L) at the Fermentation Research Institute Agency of Industrial Science and Technology (FRI), 1-3, Higashi 1-chome, Tsukuba-shi, Ibaraki-ken 305, Japan, under the terms of the Budapest Treaty on 19th August 1988 under No. FERM BP-2005.

Plasmid pIRF31 was similarly transfected into E.coli MC1061/p3 which was deposited as E.coli MC106/p3 (pIRF-31) at the FRI under the Budapest Treaty on 19th August 1988 under No. FERM BP-2006.

Regulation of the IRF gene

1. Expression of IRF-I mRNA

In view of the fact that IRF-I manifests affinities to regulatory sequences of genes other than IFN-$\beta$ gene and is thus involved in the regulation of a set of genes in various cell types, examination of the expression of the IRF-I mRNA in mouse cells derived from various tissues and organs was carried out using the murine cDNA as a probe. To prepare this probe M13 mp10 phage DNA (see below) containing the sense strand of IRF-I gene PstI fragment was used as a template to synthesise the [32]p-labeled antisense DNA, the product was digested by EcoRI and the probe DNA isolated as described by Fujita et al. (1985).

Total RNA was isolated by the established procedure of Aruffo and Seed 1987.

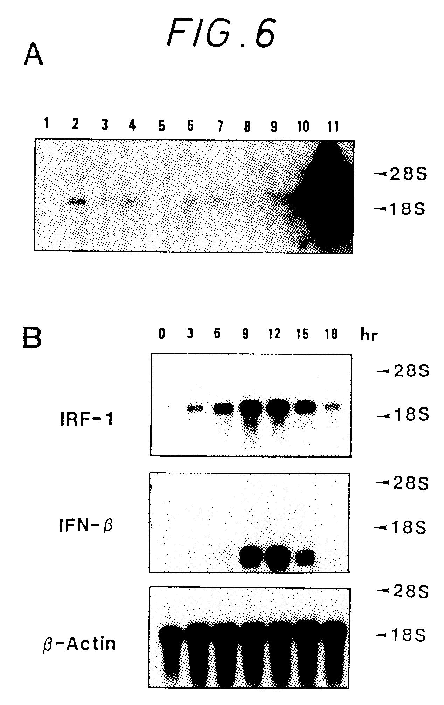

The blotting analysis was then carried out essentially as described by Thomas (1980), the x-ray film being exposed for 3 days and the results are shown in Fig. 6A. The various lanes represent the results of runs carried out using whole cell RNA from the following tissue:

Lane 1      Brain
Lane 2      Heart
Lane 3      Liver
Lane 4      Lung
Lane 5      Spleen (unstimulated)
Lane 6      Thymus
Lane 7      Kidney
Lane 8      Muscle
Lane 9      Intestine
Lane 10     Spleen (unstimulated)
Lane 11     ConA - stimulated spleen

In the run for each lane 5 $\mu$g of whole cell RNA was used, except in lane 8 for which only 1.2 $\mu$g RNA was used.

It will be seen from Fig. 6A that a band corresponding to about 2.0 kb was detected in most of the RNA samples by this blotting analysis, although the mRNA expression level seems low. It is noteworthy that the mRNA expression level in the spleen-derived lymphocytes was augmented dramatically following stimulation by ConA (Lane 11).

In a further assay mouse L929 cells were induced by NDV as described previously (Fujita et al., 1985) and the cytoplasmic RNA extracted, by the procedure of Aruffo and Seed 1987, every three hours after infection.

Probe DNAs were prepared from the following various sequences and labeled by the multiprime labeling reaction (Amersham), namely

(i) an 1.8 kb EcoRI fragment from λL28-8 (specific activity 2x10$^8$ cpm/$\mu$g);

(ii) a 0.5 kb BamHI-BglII fragment from a mouse IFN-$\beta$ genomic clone (specific activity 5x10$^8$ cpm/$\mu$g) and

(iii) a 2.0 kb BamHI-PvuII fragment of a clone containing human $\beta$-actin pseudogene (specific activity 5x10$^8$ cpm/$\mu$g).

The results are shown in Fig. 6B. Blotting analysis was carried out, as described above, using the procedure of Thomas (1980).

Each lane received 10 $\mu$g of the cytoplasmic RNA. The x-ray film was exposed for 3 hours. Densitometric analysis revealed that IRF-I mRNA increased about 25-fold, 9-12 hours after NDV infection.

Whilst the increase in mRNA is dramatic it is transient, peaking at 9 to 12 hours and levelling off 15 hours after induction. mRNA accumulation preceeds the accumulation of the IFN-B mRNA; as can be seen from Fig. 6B the induction of the IRF-I mRNA can be observed already 3 hours after NDV infection, while the IFN-$\beta$ mRNA is detectable only after 6 hours under similar blotting conditions for both RNAs.

## The IRF-I promoter

As demonstrated above, the IRF-I gene is transcriptionally regulated by various agents such as viruses and mitogenes.

Southern blot analysis of the chromosomal DNA indicated that the IRF-I gene may be spliced and not multimembered in the mouse.

An $\lambda$phage library containing new-born mouse DNA was screened for the clones harboring the IRF-I promoter sequence using the same $\lambda$L28-8 derived cDNA probe used above. Four positive clones were identified, all of which were found to contain the same genomic DNA and one of them $\lambda$g14-2 was used for further analysis. A PstI fragment was sub-cloned into the PstI site of pUC19 to construct p19IRFP.

The same DNA was thereafter cloned into the PstI site of M13mp10 and M13mp11 which were used to generate DNA for sequence analysis.

Nucleotide sequence analysis of the PstI fragment from the above clones was carried out as previously described. Major and minor CAP sites were identified by SI mapping analysis.

The determined sequence is shown in Figure 7A. As can be seen the downstream sequence of the DNA perfectly matches that of the pIRF-L derived cDNA.

The S1 nuclease analysis indicates the presence of two CAP sites for the IRF-I mRNA in which the major site is about 20 nucleotides downstream of the minor site. Typical TATA box sequences are not present within the upstream region of the gene. In view of the unusual abundancy of CpG sequence, this region probably constitutes an "HTF island" (Bird, 1986).

The promoter region contains two GC boxes and one CAAT box (see Figure 7A); the former boxes should bind SpI (Kadogan et al., 1986) and the latter, CP-1 or CP-2 (Chodosh et al., 1988).

The PstI fragment containing the promoter sequences was then tested for its reactivity in response to extracellular signals e.g. virus inducibility in the following manner:

A chimeric gene was constructed in which a reporter gene, namely a bacterial chloramphenicol acetyltransferase (CAT) gene was abutted downstream of the PstI segment. This was done by excising a PstI fragment from P19IRFP (see above) by BamHI and HindIII and cloning the resulting fragment into the BglII-HindIII backbone fragment of pA$_{10}$cat$_2$ (Rosenthal et al., 1983) to construct pIRFcat.

Several further constructs were prepared as follows:

pIRF$\Delta$ cat was prepared by digesting the p19IRFP-derived BamHI-HindIII fragment with HaeIII whose single recognition site is located at -30 to -35 from the major CAP site (Fig. 5A). The resulting HaeIII-HindIII fragment was ligated with the BglII-HindIII backbone fragment of pA$_{10}$cat$_2$ and the following synthetic DNA

$$5'GATCCTAGATTTCTTCGCGGCGC\ 3'$$

$$3'GATCTAAAGAAGCGC\ 5'$$

Thus both pIRFcat and pIRF$\Delta$ cat contained sequences up to -320 and -48 from the major CAP site respectively.

p-125cat contains the promoter sequence of the human IFN-$\beta$ gene as described by Fujita et al., 1987.

pSV2cat is described in Gorman et al., Science, Vol. 221, pp 551-553.

As a reference gene pRSVgpt was used (see Gorman et al., above).

The various genes were transfected into mouse L929 cells using the calcium phosphate method (Fujita et al., 1985). 5x10$^6$ cells were transfected with 7.5 $\mu$g of the test plasmid containing the CAT reporter gene and 2.5 $\mu$g of pRSVgpt. The cells were induced by NDV or mock-induced and then subjected to the enzyme assay as described by Fujita et al., 1985.

In calculating the relative CAT activity, CAT activity from the mock-induced cells, transfected with pSV2cat was taken as 100 %. Each CAT activity was normalised by the Ecogpt (Mulligan and Berg, Proc. Natl. Acad. Sci. USA, Vol. 78, pp 2072-2076) of the respective samples. In samples where the CAT was below the background level, they were marked as b.b..

The results are shown in Fig. 7B.

It will be seen that transfection of the pIRFcat into mouse L929 gave rise to the expression of low level CAT activity. The CAT expression level was increased when the transfected cells were stimulated by NDV. Deletion of the 300 bp upstream sequence of the IRF-I gene (pIRFΔcat) virtually abolished both constitutive and induced expression of the CAT gene. This demonstrates that the promoter sequence lies within the 300 bp upstream region and is virus inducible.

Construction of expression plasmids

1. Phage DNA of clone λL28-8 was digested by EcoRI and the cDNA insert was recovered. The EcoRI sites of the cDNA were rendered flush by T4 DNA polymerase and then ligated with synthetic adaptor DNAs having the sequence pGATCCATTGTGCTGG and pCCAGCACAATG according to Aruffo and Seed, 1987.

After removal of the synthetic DNAs by 5-20 % potassium acetate gradient centrifugation (Aruffo and Seed, 1987), the IRF-I cDNA with the adaptor DNAs attached to both its ends was ligated with BstXI-cleaved CDM8 vector DNA (Seed, B, Nature, Vo. 329, pp 840-842, 1987). Plasmids pIRF-S and pIRF-A containing the IRF-I cDNA in the sense and antisense orientation with respect to the CMV promoter respectively were isolated.

Each plasmid DNA was co-transfected with either p-55cat or p55C1B (Fujita et al., 1987) into L929 cells and the CAT expression level was determined.

The results are shown in Table 1 below:

Table 1

|  | Transfected plasmids | Induction by NDV | CAT activity (% conversion) |
|---|---|---|---|
| Exp. 1 | pIRF-S p-55cat | - | < 1 % |
|  | pIRF-S p-55ClB | - | 50 % |
|  | pIRF-A p-55cat | - | < 1 % |
|  | pIRF-A | - | < 1 % |
| Exp. 2 | pIRF-S p-55ClB | - | 1,7 % |
|  | pIRF-S p-55ClB | + | 3,6 % |
|  | pIRF-A p-55ClB | - | < 0,1 % |
|  | pIRF-A p-55ClB | + | < 0,1 % |

The DNA transfection efficiency varies depending on the state of the recipient cells (in this case, mouse L929 cells). The efficiency was much lower in Exp. 2 compared to Exp. 1. Therefore, the CAT expression level is relatively lower in Exp. 2.

As can be seen from the above table significant CAT activity was detectable only in the cells transfected by p-55ClB and pIRF-S. They demonstrate that the IRF-I binds to the repeated (8 times) AAGTGA sequences present in the upstream of the CAT gene in p-55ClB and thereby promotes transcription of the distal CAT gene.

The results further show that the CAT expression level is increased (more than two fold) by infecting the transfected cells with NDV (Table 1), demonstrating that it is possible to control the gene expression by various stimuli such as viruses.

2. An expression plasmid for the production of a protein consisting of IRF-I DNA binding domain and transcriptional activation domain of yeast GAL4 was constructed as follows:

Plasmid pIRF-S was digested by HindIII and PstI and the cDNA insert isolated. The cDNA was digested by DraIII and the HindIII-DraIII fragment (about 550 bp) was recovered and designated Fragment A.

The expression vector CDM8 was digested by HindIII and XbaI and the backbone DNA isolated and designated Fragment B.

The DNA encoding the yeast GAL4 transcriptional activation domain was isolated from plasmid pRB968 (Ma and Ptashne, 1987) as follows:

The pRB968 DNA was first digested by HindIII and the termini were rendered flush by T4 DNA polymerase. Synthetic XbaI linker DNA was added to the DNA and the DNA was subsequently digested by PvuII and XbaI.

The resulting ca. 600 bp PvuII-XbaI DNA fragment was recovered and designated Fragment C.

In addition, a synthetic DNA with the following sequence was prepared:

$$(5') GTGTCGTCCAG(3')$$

$$(3') GCACACAGCAGGTC(5')$$

designated Fragment D.

An expression vector pIRFGAL4 was constructed by ligating the Fragments A, B, C and D. As a control plasmid, plasmid pIRFΔ GAL4 was constructed by ligating Fragments A, B, C and a synthetic DNA with the following sequence:

$$(5') GTGTCTGACAG(3')$$

$$(3') GCACACAGACTGTC(5')$$

As a terminator triplet, TGA, is present in frame between the IRF-I and GAL4 sequences in pIRFΔ GAL4, the expressed protein should lack the GAL4 activation domain.

In order to test the functional properties of the plasmid encoded chimeric transcriptional factor PIRFGAL4 and pIRFΔGAL4 were each co-transfected with p-55CIB into L929 cells and CAT expression monitored. The results are shown in Table 2 below:

Table 2

| Transfected plasmid | CAT activity (% conversion) |
|---|---|
| pIRF-S p-55CIB | 2,0 % |
| pIRF-A p-55CIB | < 0,2 % |
| pIRFGAL 4 p-55CIB | 1,4 % |
| pIRFΔGAL4 p-55CIB | < 0,2 % |
| Host cell, Mouse L929 cells. Cells were not induced by NDV. | |

The results show that the expression of a target gene (such as a genes encoding an interleukin, an interferon ($\alpha$, $\beta$ and $\gamma$), a plasminogen activator, erythropoietin, granulocyte colony stimulating factor, insulin, human growth hormone or superoxide dismutase (or mutants of the human genes) can be augmented by IRF-I.

Target genes as mentioned above, such as interferon genes e.g. IFN-$\alpha$, IFN-$\beta$, IFN-$\gamma$, IFN-omega and plasminogen activators e.g. t-PA, pro-urokinase or urokinase etc. can be expressed more efficiently also by including therefor promoters fused with various lengths of recognition sequences for IRF I, e.g. AAGTGA.

For example the target genes can be introduced into various host cells, together with either intact IRF-I or chimeric IRF-I genes. By increasing the length of IRF-I recognition site DNA e.g. by increasing the number of AAGTGA repeats and the expression level of the transcription factor a high-level expression of the target genes can be achieved.

For example the AAGTGA repeat sequences can be abutted to a suitable promoter such as IFN-$\beta$ promoter or SV40 virus early promoter. A target gene e.g. a t-PA gene or IFN-$\beta$ gene can be linked downstream of such a promoter; the structure of such a constructed gene would be

$(AAGTGA)_x$ (Promoter) (target gene e.g. t-PA gene).

Such a gene could then be introduced into and amplified in CHO cells e.g. CHO DXBII (dhfr-strain) cells (Urlaub and Chasin, Proc. Natl., Acad. Sci., USA, Vol. 77, pp 4216-4220, 1980).

EP 0 359 998 B1

Ideally, as discussed above, a host cell will be chosen From a cell which has no or substantially no level of endogenous IRF-I activity. The IRF-I gene, preferably either with a strong promoter such as CMV promoter or an inducible promoter such as metallothionen gene promoter can be introduced into the various host cells in a conventional manner.

The IRF-I gene can be co-introduced and amplified together with the target gene. Alternatively the IRF-I gene and the target gene can be separately introduced into the host cell.

In such transfected cells, IRF-I may be produced either constitutively (in the case of e.g. CMV promoter) or in an induced manner (in the case of e.g. the metallothionen promoter it is induced by divalent metals such as zinc). The expressed IRF-I binds to the AAGTGA repeats and augments the distal target gene e.g. t-PA gene or IFN gene.

Such expression could be further augmented by virus e.g. NDV induction, as can be seen from Table 1, Experiment 2 such induction increases the activity of the IRF-I.

cDNA from the yeast genome which hybridises with human IRF-I cDNA sequence

Yeast DNA was prepared by the standard procedure and digested with EcoRI. 5 $\mu$g of the digested DNA was loaded onto 0,8 % agarose gel and subjected to electrophoresis and DNA blotting by standard procedures.

The blotted filter was treated exactly as described in the preceding example for isolating mouse DNA which hybridises with murine IRF-I, except as follows:

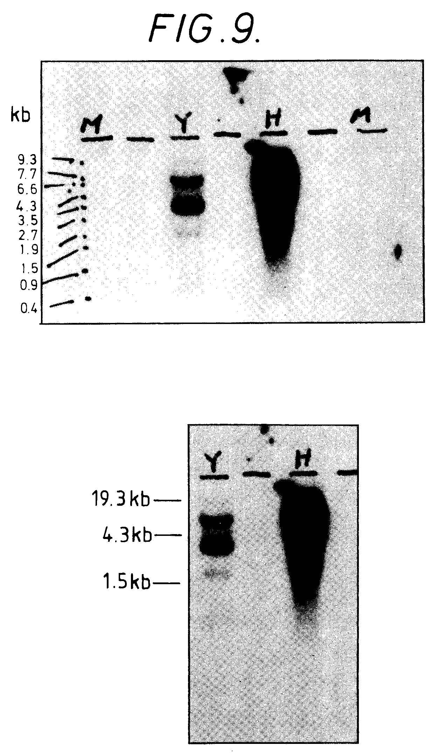

In step (3) the incubation temperature was 55°C and in step (4) the incubation was also carried out at 55°C and the radioactive probe was the human IRF-I cDNA isolated from pHIRF31 by XhoI digestion of the plasmid, this probe being labeled as described in the preceding example for murine IRF-I. The filter was washed at 55°C in 2xSSC. The positive clones were identified by autoradiography (see Figure 9).

References

Abreu S.L., Bancroft F.C., and Stewart II E.W. (1979). Interferon priming.
J. Biol. Chem. 254, 414-418.

Aruffo A., and Seed B. (1987). Molecular cloning of a cDNA by a high-efficiency COS cell expression system.
Proc. Natl. Acad. Sci. USA 84, 8573-8577.

Bird A.P. (1986). CpG-rich islands and the function of DNA methylation.
Nature 321, 209-213.

Caput D., Beutler B., Hartog K., Thayer R., Brown-Schimer S., and Cerami A. (1986). Identification of a common nucleotide sequence in the 3′ untranslated region of mRNA molecules specifying inflammatory mediators.
Proc. Natl. Acad. Sci USA 83, 1670-1674.

Cavalieri R.L., Havell E.Z., Vilcek J., and Pestka S. (1977). Induction and decay of human fibroblast interferon mRNA.
Proc. Natl. Acad. Sci. USA 74, 4415-4419.

Chodosh L.A., Baldwin A.S., Carthew R.W., and Sharp P.A. (1988). Human CCAAT-binding proteins have heterologous subunits.
Cell 53, 11-24.

Dinter H., Hauser H., Mayr U., Lammers R., Bruns W., Gross G., and Collins J. (1983). Human interferon-beta and co-induced genes: molecular studies. In the biology of the Interferon System 1983, E. De Maeyer and H. Schellekens, eds.
(Amsterdam: Elsevier Science Publishers), 33-34.

Dinter H., and Hauser H. (1987). Cooperative interaction of multiple DNA elements in the human interferon-$\beta$ promoter.
Eur. J. Biochem. 166, 103-109.

Evans R.M., and Hollenberg S.M. (1988). Zinc Fingers: Gilt by association.
Cell 52, 1-3.

Fujita T., Saito S., and Kohno S. (1979). Priming increases the amount of interferon mRNA in poly(rI)-:poly(rC)-treated L cells.
J. Gen. Viol. 45,301-308.

Fujita T., Ohno S., Yasumitsu H., and Taniguchi T. (1985). Delimination and properties of DNA sequences required for the regulated expression of human interferon-$\beta$ gene

Cell 41, 489-496.

Fujita T., Shibuya H., Hotta H., Yamanishi K., and Taniguchi T. (1987). Interfero-β gene regulation: Tandemly repeated sequences of a synthetic 6 bp oligomer function as a virus-inducible enhancer.
Cell 49, 357-367.

Galabru J., and Hovanessian A.G. (1985). Two interferon-β indeced proteins are involved in the protein kinase complex dependent on double-stranded RNA.
Cell 43, 685-694.

Goodbourn S., Zinn K., and Maniatis T. (1985). Human β-interferon gene expression is regulated by an inducible enhancer element.
Cell 41, 509-520.

Huynh T.V., Young R.A., and Davis R.W. (1985). Constructing and screening cDNA libraries in gt10 and 11. In DNA cloning-A Practical Approach, Volume 1, D.M. Glover, ed.
(Oxford: IRL Press), pp. 49-78.

Israel A., Kimura A., Fournier A., Fellous M., and Kourilsky P. (1986). Interferon response sequence potentiates activity of an enhancer in the promoter region of a mouse H-2 gene.
Nature 322, 743-746.

Kadonaga J.T., Jones K.A., and Tjian R. (1986). Promoter-specific activation of RNA polymerase II transcription by Sp 1.
Trends Biochem. Sci. 11, 20-23.

Kakidani H., and Ptashne M. (1986). GAL4 activates gene expression in mammalian cells.
Cell 52, 161-167.

Keller A.D., and Maniatis T. (1988). Identification of an inducible factor that binds to a positive regulatory element of the human β-interferon gene.
Proc. Natl. Acad. Sci USA 85, 3309-3313.

Kohase M., May L.T., Tamm I., Vilcek J., and Sehgal P.B. (1987). A cytokine network in human diploid fibroblasts: interactions of beta interferons, tumor necrosis factor, platelet-derived growth factor and interleukin-1. Mol.
Cell. Biol. 7, 272-280.

Korber B., Mermod N., Hood L., and Stroynowski I. (1988). Regulation of gene expression by interferons: Control of H-2 Promoter responses.
Science 239, 1302-1306.

Kozak M. (1987). An analysis of 5′-noncoding sequences from 699 vertebrate messenger RNAs.
Nucl. Acids Res. 15, 8125-8143.

Krebs E., Eisenman R., Kuenzel E., Litchfield D., Lozeman F., Liischer B., and Sommercorn J. (1988). Casein Kinase II as a potentially important enzyme concerned with signal transduction. In the Molecular Biology of Signal Transduction (Cold Spring Harbor, New York: Cold Spring Harbor Laboratory) Abstract p.35.

Kuhl D., de la Fuente J., Chaturvedi M., Parimoo S., Ryals J., Mayer F., and Weissmann C. (1987). Reversible silencing of enhancers by sequences derived from the human IFN-α promoter.
Cell 50, 1057-1069.

Kyte J., and Doolittle R.F. (1982). A simple method for displaying the hydropathic character of a protein.
J. Mol. Biol. 157, 105-132.

Levy D.E., Kessler D.S., Pine R., Reich N., and Darnell J.E. (1988). Interferon-induced nuclear factors that bind a shared promoter element correlate with positive and negative transcriptional control.
Genes and Development 2, 383-393.

Ma J., and Ptashne M. (1987). The carboxy-terminal 30 amino acids of GAL4 are recognised by GAL80.
Cell 50, 137-142.

Maxam A., and Gilbert W. (1980). Sequencing end-labeled DNA with base specific chemical cleavages.
Meth. Enzym. 65, 499-560.

Moore R.N., Larsen H.S., Horohov D.W., and Rouse B.T. (1984). Endogenous regulation of macrophase proliferative expansion by colony-stimulation-factor-induced interferon.
Science 223, 178-180.

Nedwin G., Naylor S., Sakaguchi A., Smith D., Jarrett-Nedsin J., Pennica D., Goeddel D., and Gray P. (1985). Human lymphotoxin and tumor necrosis factor genes: structure, homology and chromosomal localisation.
Nucl. Acids Res. 13, 6361-6373.

Nir U., Cohen B., Chen L., and Revel M. (1984). A human IFN-β1 gene deleted of promoter sequences upstream from the TATA box is controlled post-transcriptionally by dsRNA.

Nucl. Acids Res. 12, 6979-6993.

Ohno S., and Taniguchi T. (1983). The 5'-flanking sequence of human interferon-β gene is responsible for viral induction of transcription.
Nucl. Acids Res. 11, 5403-5412.

Onozaki K., Urawa H., Tamatani T., Iwamura Y., Hashimoto T., Baba T., Suzuki H., Yamada M., Yamamoto S., Oppenheim J.J., and Matsushima K. (1988).Synergistic interactions of interleukin 1, interferon-β and tumor necrosis factor in terminally differentiating a mouse myeloid leukemic cell line M1).
J. Immunol. 140, 112-119.

Pabo C.O., and Sauer R.T. (1984). Protein-DNA recognition.
Ann. Rev. Biochem. 53, 293-321.

Raji N.B.K., and Pitha P.M. (1981). An analysis of interferon mRNA in human fibroblast cells induced to produce interferon.
Proc. Natl. Acad. Sci USA 78, 7426-7430.

Raji N.B.K., and Pitha P.M. (1983). Two levels of regulation of β-interferon gene expression in human cells.
Proc. Natl. Acad. Sci USA 80, 3923-3927.

Resnitzky D., Yarden A., Zipori D., and Kimchi A. (1986). Autocrine β-related interferon controls c-myc suppression and growth arrest during hematopoietic cell differentiation.
Cell 46, 31-40.

Rosenthal N., Kress M., Gruss P., and Khoury G. (1983). BK viral enhancer element and a human cellular homolog.
Science 222, 749-755.

Ryals J., Dieks P., Ragg H., and Weissmann C. (1985). A 46-nucleotide promoter segment from an INF-α gene renders an unrelated promoter inducible by virus.
Cell 41, 497-507.

Shaw G., and Kamen R. (1986). A conserved AU sequence from the 3' untranslated region of GM-CSF mRNA mediates selective mRNA degradation.
Cell 46, 659-667.

Singh H., LeBowitz J.H., Baldwin Jr.A.S., and Sharp P.A. (1988). Molecular cloning of an enhancer binding protein: Isolation by screening of an expression library with a recognition site DNA.
Cell 52 415-423.

Sugita K., Miyazaki J.I., Appella E., and Ozato K. (1987). Interferons increase transcription of a major histocompatibility class I gene via a 5' interferon consensus sequence.
Mol. Cell. Biol. 7, 2625-2630.

Taniguchi T., Matsui H., Fujita T., Takaoka C., Kashima N., Yoshimoto R., and Hamuro J. (1983). Structure and expression of a cloned cDNA for human interleukin-2.
Nature 302, 305-310.

Taniguchi T. (1988). regulation of cytokine gene expression.
Ann. Rev. Immunol. 6, 439-464.

Thomas P.S. (1980). Hybridisation of denatured RNA and small DNA fragments transferred to nitrocellulose.
Proc. Natl. Acad. Sci. USA 77, 5201-5205.

Tiwari R.J., Kusari J., and Sen G.C. (1987). Functional equivalents of interferon-mediated signals needed for induction of a mRNA can be generated by double-stranded RNA and growth factors.
EMBO J. 6, 3373-3378.

Warren M.K., and Ralf P. (1986). Macrophage growth factor CSF-1 stimulates human monocyte production of interferon, tumor necrosis factor and colony stimulating activity.
J. Immunol. 137, 2281-2285.

Webster N., Jin J.R., Green S., Hollis M., and Chambon P. (1988). The yeast UAGs is a transcriptional enhancer in human HeLa cells in the presence of the GAL4 trans-activator.
Cell 52, 169-178.

Weissmann C., and Weber H. 81986). The interferon genes.
Proc. Natl. Acid Res. Mol. Biol., 33, 251-300.

Young R.A., and Davis R.W. (1983). Yeast RNA polymerase II genes: Isolation with antibody probes.
Science 222, 778-782.

Zinn K., Dimaio D., and Maniatis T. (1983). Identification of two distinct regulatory regions adjacent to the human β-interferon gene.
Cell 34, 865-879.

Zullo J.N., Cochan B.H., Huang A.S., and Stiles C.D. (1985). Platelet-derived growth factor and double-stranded ribonucleic acids and stimulate expression of the same genes in 3T3 cells.
Cell 43, 793-800.

**Claims**

1. A recombinant DNA molecule characterised by a structural gene having the formula

```
          10        20        30        40        50
ATGCCCATCACTTGGATGCGCATGAGACCCTGGCTAGAGATGCAGATTAA

          60        70        80        90       100
TTCCAACCAAATCCCGGGGCTCATCTGGATTAATAAAGAGGAGATGATCT

         110       120       130       140       150
TGGAGATCCCATGGAAGCATGCTGCCAAGCATGGCTGGGACATCAACAAG

         160       170       180       190       200
GATGCCTGTTTGTTCCGGAGCTGGGCCATTCACACAGGCCGATACAAAGC

         210       220       230       240       250
AGGGGAAAAGGAGCCAGATCCCAAGACGTGGAAGGCCAACTTTCGCTGTG

         260       270       280       290       300
CCATGAACTCCCTGCCAGATATCGAGGAGGTGAAAGACCAGAGCAGGAAC

         310       320       340       340       350
AAGGGCAGCTCAGCTGTGCGAGTGTACCGGATGCTTCCACCTCTCACCAA

         360       370       380       390       400
GAACCAGAGAAAAGAAAGAAAGTCGAAGTCCAGCCGAGATGCTAAGAGCA

         410       420       430       440       450
AGGCCAAGAGGAAGTCATGTGGGGATTCCAGCCCTGATACCTTCTCTGAT

         460       470       480       490       500
GGACTCAGCAGCTCCACTCTGCCTGATGACCACAGCAGCTACACAGTTCC

         510       520       530       540       550
AGGCTACATGCAGGACTTGGAGGTGGAGCAGGCCCTGACTCCAGCACTGT

         560       570       580       590       600
CGCCATGTGCTGTCAGCAGCACTCTCCCCGACTGGCACATCCCAGTGGAA

         610       620       630       640       650
GTTGTGCCGGACAGCACCAGTGATCTGTACAACTTCCAGGTGTCACCCAT

         660       670       680       690       700
GCCCTCCATCTCTGAAGCTACAACAGATGAGGATGAGGAAGGGAAATTAC

         710       720       730       740       750
CTGAGGACATCATGAAGCTCTTGGAGCAGTCGGAGTGGCAGCCAACAAAC

         760       770       780       790       800
GTGGATGGGAAGGGGTACCTACTCAATGAACCTGGAGTCCAGCCCACCTC
```

```
          810       820       830       840       850
TGTCTATGGAGACTTTAGCTGTAAGGAGGAGCCAGAAATTGACAGCCCAG

          860       870       880       890       900
GGGGGGATATTGGGCTGAGTCTACAGCGTGTCTTCACAGATCTGAAGAAC

          910       920       930       940       950
ATGGATGCCACCTGGCTGGACAGCCTGCTGACCCCAGTCCGGTTGCCCTC

          960       970
CATCCAGGCCATTCCCTGTGCACCG
```

or

```
ATG CCA ATC ACT CGA ATG CGG ATG AGA CCC TGG CTA GAG ATG CAG ATT
AAT TCC AAC CAA ATC CCA GGG CTG ATC TGG ATC AAT AAA GAA GAG ATG
ATC TTC CAG ATT CCA TGG AAG CAC GCT GCT AAG CAC GGC TGG GAC ATC
AAC AAG GAT GCC TGT CTG TTC CGG AGC TGG GCC ATT CAC ACA GGC CGA
TAC AAA GCA GGA GAA AAA GAG CCA GAT CCC AAG ACA TGG AAG GCA AAC
TTC CGT TGT GCC ATG AAC TCC CTG CCA GAC ATC GAG GAA GTG AAG GAT
CAG AGT AGG AAC AAG GGC AGC TCT GCT GTG CGG GTG TAC CGG ATG CTG
CCA CCC CTC ACC AGG AAC CAG AGG AAA GAG AGA AAG TCC AAG TCC AGC
CGA GAC ACT AAG AGC AAA ACC AAG AGG AAG CTG TGT GGA GAT GTT AGC
CCG GAC ACT TTC TCT GAT GGA CTC AGC AGC TCT ACC CTA CCT GAT GAC
CAC AGC AGT TAC ACC ACT CAG GGC TAC CTG GGT CAG GAC TTG GAT ATG
GAA AGG GAC ATA ACT CCA GCA CTG TCA CCG TGT GTC GTC AGC AGC AGT
CTC TCT GAG TGG CAT ATG CAG ATG GAC ATT ATA CCA GAT AGC ACC ACT
GAT CTG TAT AAC CTA CAG GTG TCA CCC ATG CCT TCC ACC TCC GAA GCC
GCA ACA GAC GAG GAT GAG GAA GGG AAG ATA GCC GAA GAC CTT ATG AAG
CTC TTT GAA CAG TCT GAG TGG CAG CCG ACA CAC ATC GAT GGC AAG GGA
TAC TTG CTC AAT GAG CCA GGG ACC CAG CTC TCT TCT GTC TAT GGA GAC
TTC AGC TGC AAA GAG GAA CCA GAG ATT GAC AGC CCT CGA GGG GAC ATT
GGG ATA GGC ATA CAA CAT GTC TTC ACG GAG ATG AAG AAT ATG GAC TCC
ATC ATG TGG ATG GAC AGC CTG CTG GGC AAC TCT GTG AGG CTG CCG CCC
TCT ATT CAG GCC ATT CCT TGT GCA CCA TAG
```

or a fragment, or a degenerate variant of the molecule or fragment, which codes for a protein having the activity of an interferon regulatory factor-1 (IRF-1).

2. A recombinant DNA sequence which hybridises to the anti-sense version of a molecule as defined in claim 1 and which codes for a protein having IRF-1 activity.

3. A recombinant DNA molecule according to claim 1 or 2, comprising a promoter and regulator sequence contained within the following formula:

```
        PstI
        CTGCAGAAAGAGGGGGACGGTCTCGGCTTTCCAAGACAGGCAAGGGGG
        -299


                            GC Box 1                    GC Box 2
CAGGGGAGTGGAGTGGAGCAAGGGGCGGGCCCGCGGTAGCCCCGGGGCGGTGGCGCGG
-251


GCCCGAGGGGGTGGGGAGCACAGCTGCCTTGTACTTCCCCTTCGCCGCTTAGCTCTAC
-193


                                    CAAT Box
AACAGCCTGATTTCCCCGAAATGATGAGGCCGAGTGGGCCAATGGGCGCGCAGGAGCG
-135


                                                Minor Cap site
                                                    ▼▼▼
GCGCGGCGGGGGCGTGGCCGAGTCCGGGCCGGGGAATCCCGCTAAGTGTTTAGATTTC
-77                                                          -21

        Major Cap site                         ___pIRF-L
            ▼▼▼
TTCGCGGCGCCGCGGACTCGCCAGTGCGCACCACTCCTTCGTCGAGGTAGGACGTGCT
-19                 +1


TTCACAGTCTAAGCCGAACCGAACCGAACCGAACCGAACCGAACCGGGCCGAGTTGCG
+39


CCGAGGTCAGCCGAGGTGGCCAGAGGACCCCAGCATCTCGGGCATCTTTCGCTTCGTG
+97


CGCGCATCGCGTACCTACACCGCAACTCCGTGCCTCGCTCTCCGGCACCCTCTGCGAA
+155
        PstI
TCGCTCCTGCAG
+213        +225
```

4. A recombinant DNA molecule comprising a DNA sequence as defined in any one of claims 1 to 3 and a structural gene for a desired pharmaceutically active protein under the control of the IRF-I protein coded for by said DNA sequence.

5. A recombinant DNA molecule as defined in claim 4, wherein the gene coding for the IRF-I active molecule is under the control of a constitutive promoter or an inducible promoter.

6. A recombinant DNA molecule as defined in claim 4 or 5 wherein the gene for the desired pharmaceutically active protein includes an IRF-I binding site containing a plurality of AAGTGA repeat sequences.

7. A recombinant DNA molecule as defined in any one of claims 4 to 6 wherein the desired, pharmaceutically active protein is a cytokine or a plasminogen activator.

8. A host cell transformed by a recombinant DNA molecule as defined in any one of Claims 1 to 7.

9. A host cell as defined in claim 8, which has been transformed by a first DNA molecule containing a sequence coding for a protein having the activity of an IRF-I, and by a second separate DNA molecule containing a gene coding for a desired, pharmaceutically active protein under the control of the IRF-I protein coded for by the first DNA molecule.

10. A host cell as defined in claim 9, wherein the gene coding for the IRF-I active molecule is under the control of a constitutive promoter or an inducible promoter.

11. A host cell as defined in claim 8 or 9 to 10 wherein the gene for the desired pharmaceutically active protein includes a binding site for the IRF-I active protein which includes a plurality of AAGTGA repeat sequences.

12. A transformed host cell as defined in anyone of claims 8 to 11 which is a bacterial cell or a yeast cell or a mammalian cell.

13. A method of producing a desired, pharmaceutically active protein which comprises cultivating host cells as defined in anyone of claims 8 to 12.

14. A protein having the structure defined by a structural gene as claimed in claim 1 or 2.

15. A plasmid, which is
Plasmid pHIRF31, FERM BP-2006;
Plasmid pIRF-L, FERM BP-2OO5; or
Plasmid pIRF2-5, FERM BP-2157.

**Patentansprüche**

1. Rekombinantes DNA-Molekül, gekennzeichnet durch ein Strukturgen mit der Formel

```
          10        20        30        40        50
ATGCCCATCACTTGGATGCGCATGAGACCCTGGCTAGAGATGCAGATTAA

          60        70        80        90       100
TTCCAACCAAATCCCGGGGCTCATCTGGATTAATAAAGAGGAGATGATCT

         110       120       130       140       150
TGGAGATCCCATGGAAGCATGCTGCCAAGCATGGCTGGGACATCAACAAG

         160       170       180       190       200
GATGCCTGTTTGTTCCGGAGCTGGGCCATTCACACAGGCCGATACAAAGC

         210       220       230       240       250
AGGGGAAAAGGAGCCAGATCCCAAGACGTGGAAGGCCAACTTTCGCTGTG

         260       270       280       290       300
CCATGAACTCCCTGCCAGATATCGAGGAGGTGAAAGACCAGAGCAGGAAC

         310       320       340       340       350
AAGGGCAGCTCAGCTGTGCGAGTGTACCGGATGCTTCCACCTCTCACCAA

         360       370       380       390       400
GAACCAGAGAAAAGAAAGAAAGTCGAAGTCCAGCCGAGATGCTAAGAGCA

         410       420       430       440       450
AGGCCAAGAGGAAGTCATGTGGGGATTCCAGCCCTGATACCTTCTCTGAT

         460       470       480       490       500
GGACTCAGCAGCTCCACTCTGCCTGATGACCACAGCAGCTACACAGTTCC

         510       520       530       540       550
AGGCTACATGCAGGACTTGGAGGTGGAGCAGGCCCTGACTCCAGCACTGT

         560       570       580       590       600
CGCCATGTGCTGTCAGCAGCACTCTCCCCGACTGGCACATCCCAGTGGAA

         610       620       630       640       650
GTTGTGCCGGACAGCACCAGTGATCTGTACAACTTCCAGGTGTCACCCAT

         660       670       680       690       700
GCCCTCCATCTCTGAAGCTACAACAGATGAGGATGAGGAAGGGAAATTAC

         710       720       730       740       750
CTGAGGACATCATGAAGCTCTTGGAGCAGTCGGAGTGGCAGCCAACAAAC

         760       770       780       790       800
GTGGATGGGAAGGGGTACCTACTCAATGAACCTGGAGTCCAGCCCACCTC
```

```
             810         820         830         840         850
  TGTCTATGGAGACTTTAGCTGTAAGGAGGAGCCAGAAATTGACAGCCCAG

             860         870         880         890         900
  GGGGGGATATTGGGCTGAGTCTACAGCGTGTCTTCACAGATCTGAAGAAC

             910         920         930         940         950
  ATGGATGCCACCTGGCTGGACAGCCTGCTGACCCCAGTCCGGTTGCCCTC

             960         970
  CATCCAGGCCATTCCCTGTGCACCG
```

oder

```
ATG CCA ATC ACT CGA ATG CGG ATG AGA CCC TGG CTA GAG ATG CAG ATT
AAT TCC AAC CAA ATC CCA GGG CTG ATC TGG ATC AAT AAA GAA GAG ATG
ATC TTC CAG ATT CCA TGG AAG CAC GCT GCT AAG CAC GGC TGG GAC ATC
AAC AAG GAT GCC TGT CTG TTC CGG AGC TGG GCC ATT CAC ACA GGC CGA
TAC AAA GCA GGA GAA AAA GAG CCA GAT CCC AAG ACA TGG AAG GCA AAC
TTC CGT TGT GCC ATG AAC TCC CTG CCA GAC ATC GAG GAA GTG AAG GAT
CAG AGT AGG AAC AAG GGC AGC TCT GCT GTG CGG GTG TAC CGG ATG CTG
CCA CCC CTC ACC AGG AAC CAG AGG AAA GAG AGA AAG TCC AAG TCC AGC
CGA GAC ACT AAG AGC AAA ACC AAG AGG AAG CTG TGT GGA GAT GTT AGC
CCG GAC ACT TTC TCT GAT GGA CTC AGC AGC TCT ACC CTA CCT GAT GAC
CAC AGC AGT TAC ACC ACT CAG GGC TAC CTG GGT CAG GAC TTG GAT ATG
GAA AGG GAC ATA ACT CCA GCA CTG TCA CCG TGT GTC GTC AGC AGC AGT
CTC TCT GAG TGG CAT ATG CAG ATG GAC ATT ATA CCA GAT AGC ACC ACT
GAT CTG TAT AAC CTA CAG GTG TCA CCC ATG CCT TCC ACC TCC GAA GCC
GCA ACA GAC GAG GAT GAG GAA GGG AAG ATA GCC GAA GAC CTT ATG AAG
CTC TTT GAA CAG TCT GAG TGG CAG CCG ACA CAC ATC GAT GGC AAG GGA
TAC TTG CTC AAT GAG CCA GGG ACC CAG CTC TCT TCT GTC TAT GGA GAC
TTC AGC TGC AAA GAG GAA CCA GAG ATT GAC AGC CCT CGA GGG GAC ATT
GGG ATA GGC ATA CAA CAT GTC TTC ACG GAG ATG AAG AAT ATG GAC TCC
ATC ATG TGG ATG GAC AGC CTG CTG GGC AAC TCT GTG AGG CTG CCG CCC
TCT ATT CAG GCC ATT CCT TGT GCA CCA TAG
```

oder ein Fragment, oder eine degenerierte Variante des Moleküls oder des Fragmentes, das für ein Protein codiert, das die Aktivität des Interferon-Regulator-Faktors-1 (IRF-1) besitzt.

2. Rekombinante DNA-Sequenz, die mit der anti-Sense-Version eines Moleküls gemäß Anspruch 1 hybridisiert, und die für ein Protein codiert, das IRF-1-Aktivität besitzt.

3. Rekombinantes DNA-Molekül gemäß Anspruch 1 oder 2, umfassend eine Promotor- und Regulatorsequenz, die in der folgenden Formel vorkommt:

```
            PstI
        CTGCAGAAAGAGGGGGACGGTCTCGGCTTTCCAAGACAGGCAAGGGGG
        -299
```

```
                            GC Box 1                    GC Box 2
        CAGGGGAGTGGAGTGGAGCAAGGGGCGGGCCCGCGGTAGCCCCGGGGCGGTGGCGCGG
        -251
```

```
        GCCCGAGGGGGTGGGGAGCACAGCTGCCTTGTACTTCCCCTTCGCCGCTTAGCTCTAC
        -193
```

```
                                            CAAT Box
        AACAGCCTGATTTCCCCGAAATGATGAGGCCGAGTGGGCCAATGGGCGCGCAGGAGCG
        -135
```

```
                                            Schwache Cap-Site
                                                  ↑↑↑
        GCGCGGCGGGGGCGTGGCCGAGTCCGGGCCGGGGAATCCCGCTAAGTGTTTAGATTTC
        -77                                                     -21
```

```
            Starke Cap-Site                              ┌──plRF-L
                 ↑↑↑                                      │
        TTCGCGGCGCCGCGGACTCGCCAGTGCGCACCACTCCTTCGTCGAGGTAGGACGTGCT
        -19                +1
```

```
        TTCACAGTCTAAGCCGAACCGAACCGAACCGAACCGAACCGAACCGGGCCGAGTTGCG
        +39
```

```
        CCGAGGTCAGCCGAGGTGGCCAGAGGACCCCAGCATCTCGGGCATCTTTCGCTTCGTG
        +97
```

```
        CGCGCATCGCGTACCTACACCGCAACTCCGTGCCTCGCTCTCCGGCACCCTCTGCGAA
        +155
            PstI
        TCGCTCCTGCAG
        +213        +225
```

4. Rekombinantes DNA-Molekül, umfassend eine DNA-Sequenz gemäß einem der Ansprüche 1 bis 3 und ein Strukturgen für ein gewünschtes, pharmazeutisch aktives Protein unter der Kontrolle des IRF-1-Proteins, das von der DNA-Sequenz codiert ist.

5. Rekombinantes DNA-Molekül gemäß Anspruch 4, worin sich das für das IRF-1-aktive Molekül codierende Gen unter der Kontrolle eines konstitutiven Promotors oder eines induzierbaren Promotors befindet.

6. Rekombinantes DNA-Molekül gemäß Anspruch 4 oder 5, worin das Gen für das gewünschte, pharmazeutisch aktive Protein eine IRF-1-Bindungsstelle umfaßt, die eine Vielzahl von AAGTGA-Sequenzwiederholungen enthält.

7. Rekombinantes DNA-Molekül gemäß einem der Ansprüche 4 bis 6, worin das gewünschte, pharmazeutisch aktive Protein ein Cytokin oder ein Plasminogen-Aktivator ist.

8. Wirtszelle, transformiert mit einem rekombinanten DNA-Molekül gemäß einem der Ansprüche 1 bis 7.

**9.** Wirtszelle gemäß Anspruch 8, die transformiert worden ist mit einem ersten DNA-Molekül, das eine Sequenz enthält, die für ein Protein codiert, das die Aktivität von IRF-1 besitzt, und mit einem zweiten separaten DNA-Molekül, das ein Gen enthält, das für ein gewünschtes, pharmazeutisch aktives Protein codiert, das sich unter der Kontrolle des IRF-1-Proteins befindet, das von dem ersten DNA-Molekül codiert wird.

**10.** Wirtszelle gemäß Anspruch 9, worin sich das für das IRF-1-aktive Molekül codierende Gen unter der Kontrolle eines konstitutiven Promotors oder eines induzierbaren Promotors befindet.

**11.** Wirtszelle gemäß Anspruch 8 oder 9 bis 10, worin das Gen für das gewünschte, pharmazeutisch aktive Protein eine Bindungsstelle für das IRF-1-aktive Protein umfaßt, die eine Vielzahl von AAGTGA-Sequenzwiederholungen umfaßt.

**12.** Transformierte Wirtszelle gemäß einem der Ansprüche 8 bis 11, ausgewählt unter einer Bakterienzelle, einer Hefezelle oder einer Säugerzelle.

**13.** Verfahren zur Herstellung eines gewünschten, pharmazeutisch aktiven Proteins, wobei man eine Wirtszelle gemäß einem der Ansprüche 8 bis 12 kultiviert.

**14.** Protein, das die durch ein Strukturgen gemäß Anspruch 1 oder 2 definierte Struktur besitzt.

**15.** Plasmid, ausgewählt unter
Plasmid pHIRF31, FERM BP-2006;
Plasmid pIRF-L, FERM BP-2005; oder
Plasmid pIRF2-5, FERM BP-2157.

**Revendications**

1. Molécule d'ADN recombiné caractérisée pu un gène de structure ayant la formule

```
        10        20        30        40        50
ATGCCCATCACTTGGATGCGCATGAGACCCTGGCTAGAGATGCAGATTAA

        60        70        80        90       100
TTCCAACCAAATCCCGGGGCTCATCTGGATTAATAAAGAGGAGATGATCT

       110       120       130       140       150
TGGAGATCCCATGGAAGCATGCTGCCAAGCATGGCTGGGACATCAACAAG

       160       170       180       190       200
GATGCCTGTTTGTTCCGGAGCTGGGCCATTCACACAGGCCGATACAAAGC

       210       220       230       240       250
AGGGGAAAAGGAGCCAGATCCCAAGACGTGGAAGGCCAACTTTCGCTGTG

       260       270       280       290       300
CCATGAACTCCCTGCCAGATATCGAGGAGGTGAAAGACCAGAGCAGGAAC

       310       320       340       340       350
AAGGGCAGCTCAGCTGTGCGACTGTACCGGATGCTTCCACCTCTCACCAA

       360       370       380       390       400
GAACCAGAGAAAAGAAAGAAAGTCGAAGTCCAGCCGAGATGCTAAGAGCA

       410       420       430       440       450
AGGCCAAGAGGAAGTCATGTGGGGATTCCAGCCCTGATACCTTCTCTGAT

       460       470       480       490       500
GGACTCAGCAGCTCCACTCTGCCTGATGACCACAGCAGCTACACAGTTCC

       510       520       530       540       550
AGGCTACATGCAGGACTTGGAGGTGGAGCAGGCCCTGACTCCAGCACTGT

       560       570       580       590       600
CGCCATGTGCTGTCAGCAGCACTCTCCCCGACTGGCACATCCCAGTGGAA

       610       620       630       640       650
GTTGTGCCGGACAGCACCAGTGATCTGTACAACTTCCAGGTGTCACCCAT

       660       670       680       690       700
GCCCTCCATCTCTGAAGCTACAACAGATGAGGATGAGGAAGGGAAATTAC

       710       720       730       740       750
CTGAGGACATCATGAAGCTCTTGGAGCAGTCGGAGTGGCAGCCAACAAAC

       760       770       780       790       800
GTGGATGGGAAGGGGTACCTACTCAATGAACCTGGAGTCCAGCCCACCTC
```

```
            810        820        830        840        850
   TGTCTATGGAGACTTTAGCTGTAAGGAGGAGCCAGAAATTGACAGCCCAG

            860        870        880        890        900
   GGGCGGATATTGGGCTGAGTCTACAGCGTGTCTTCACAGATCTGAAGAAC

            910        920        930        940        950
   ATGGATGCCACCTGGCTGGACAGCCTGCTGACCCCAGTCCGGTTGCCCTC

            960        970
   CATCCAGGCCATTCCCTGTGCACCG
```

ou

```
ATG CCA ATC ACT CGA ATG CGG ATG AGA CCC TGG CTA GAG ATG CAG ATT
AAT TCC AAC CAA ATC CCA GGG CTG ATC TGG ATC AAT AAA GAA GAG ATG
ATC TTC CAG ATT CCA TGG AAG CAC GCT GCT AAG CAC GGC TGG GAC ATC
AAC AAG GAT GCC TGT CTG TTC CGG AGC TGG GCC ATT CAC ACA GGC CGA
TAC AAA GCA GGA GAA AAA GAG CCA GAT CCC AAG ACA TGG AAG GCA AAC
TTC CGT TGT GCC ATG AAC TCC CTG CCA GAC ATC GAG GAA GTG AAG GAT
CAG AGT AGG AAC AAG GGC AGC TCT GCT GTG CGG GTG TAC CGG ATG CTG
CCA CCC CTC ACC AGG AAC CAG AGG AAA GAG AGA AAG TCC AAG TCC AGC
CGA GAC ACT AAG AGC AAA ACC AAG AGG AAG CTG TGT GGA GAT GTT AGC
CCG GAC ACT TTC TCT GAT GGA CTC AGC AGC TCT ACC CTA CCT GAT GAC
CAC AGC AGT TAC ACC ACT CAG GGC TAC CTG GGT CAG GAC TTG GAT ATG
GAA AGG GAC ATA ACT CCA GCA CTG TCA CCG TGT GTC GTC AGC AGC AGT
CTC TCT GAG TGG CAT ATG CAG ATG GAC ATT ATA CCA GAT AGC ACC ACT
GAT CTG TAT AAC CTA CAG GTG TCA CCC ATG CCT TCC ACC TCC GAA GCC
GCA ACA GAC GAG GAT GAG GAA GGG AAG ATA GCC GAA GAC CTT ATG AAG
CTC TTT GAA CAG TCT GAG TGG CAG CCG ACA CAC ATC GAT GGC AAG GGA
TAC TTG CTC AAT GAG CCA GGG ACC CAG CTC TCT TCT GTC TAT GGA GAC
TTC AGC TGC AAA GAG GAA CCA GAG ATT GAC AGC CCT CGA GGG GAC ATT
GGG ATA GGC ATA CAA CAT GTC TTC ACG GAG ATG AAG AAT ATG GAC TCC
ATC ATG TGG ATG GAC AGC CTG CTG GGC AAC TCT GTG AGG CTG CCG CCC
TCT ATT CAG GCC ATT CCT TGT GCA CCA TAG
```

ou un fragment, ou une variante dégénérée de la molécule ou du fragment, qui code pour une protéine ayant l'activité d'un facteur-1 régulateur de l'interféron (IRF-1).

2. Séquence d'ADN recombiné qui s'hybride à la version antisens d'une molécule selon la revendication 1 et qui code pour une protéine ayant une activité d'IRF-1.

3. Molécule d'ADN recombiné selon la revendication 1 ou 2, comprenant une séquence promotrice et régulatrice contenue dans la formule suivante:

```
                        PstI
            CTGCAGAAAGAGGGGGACGGTCTCGGCTTTCCAAGACAGGCAAGGGGG
            -299

                              boîte GC 1                    boîte GC 2
    CAGGGGAGTGGAGTGGAGCAAGGGGCGGGCCCGCGGTAGCCCCGGGGCGGTGGCGCGG
    -251


    GCCCGAGGGGGTGGGGAGCACAGCTGCCTTGTACTTCCCCTTCGCCGCTTAGCTCTAC
    -193

                                          boîte CAAT
    AACAGCCTGATTTCCCCGAAATGATGAGGCCGAGTGGGCCAATGGGCGCGCAGGAGCG
    -135

                                                      site Cap mineur
                                                          ↓↓↓
    .GCGCGGCGGGGGCGTGGCCGAGTCCGGGCCGGGGAATCCCGCTAAGTGTTTAGATTTC
    -77                                                      -21

        site Cap majeur                              ┌───plRF-L
            ↓↓↓                                       │
    TTCGCGGCGCCGCGGACTCGCCAGTGCGCACCACTCCTTCGTCGAGGTAGGACGTGCT
    -19             +1


    TTCACAGTCTAAGCCGAACCGAACCGAACCGAACCGAACCGAACCGGGCCGAGTTGCG
    +39


    CCGAGGTCAGCCGAGGTGGCCAGAGGACCCCAGCATCTCGGGCATCTTTCGCTTCGTG
    +97

    CGCGCATCGCGTACCTACACCGCAACTCCGTGCCTCGCTCTCCGGCACCCTCTGCGAA
    +155

            PstI
    TCGCTCCTGCAG
    +213        +225
```

4. Molécule d'ADN recombiné comprenant une séquence d'ADN selon l'une quelconque des revendications 1 à 3 et un gène de structure pour une protéine pharmaceutiquement active désirée sous le contrôle de la protéine d'IRF-1 pour laquelle code ladite séquence d'ADN.

5. Molécule d'ADN recombiné selon la revendication 4, dans laquelle le gène codant pour la molécule à activité d'IRF-1 est sous le contrôle d'un promoteur constitutif ou d'un promoteur pouvant être induit.

6. Molécule d'ADN recombiné selon la revendication 4 ou 5, dans laquelle le gène codant pour la protéine pharmaceutiquement active désirée comprend un site de fixation à l'IRF-1, contenant une pluralité de séquences de répétition AAGTGA.

7. Molécule d'ADN recombiné selon l'une quelconque des revendications 4 à 6, dans laquelle la protéine pharmaceutiquement active désirée est une cytokine ou un activateur de plasminogène.

8. Cellule hôte transformée par une molécule d'ADN recombiné selon l'une quelconque des revendications 1 à 7.

**9.** Cellule hôte selon la revendication 8, qui a été transformée par une première molécule d'ADN contenant une séquence codant pour une protéine ayant l'activité d'un IRF-1, et par une seconde molécule d'ADN séparée contenant un gène codant pour une protéine pharmaceutiquement active désirée sous le contrôle de la protéine d'IRF-1 pour laquelle code la première molécule d'ADN.

**10.** Cellule hôte selon la revendication 9, dans laquelle le gène codant pour la molécule à activité d'IRF-1 est sous le contrôle d'un promoteur constitutif ou d'un promoteur pouvant être induit.

**11.** Cellule hôte selon la revendication 8 ou 9 à 10, dans laquelle le gène pour la protéine pharmaceutiquement active désirée comprend un site de fixation pour la protéine à activité d'IRF-1, qui comprend une pluralité de séquences de répétition AAGTGA.

**12.** Cellule hôte transformée selon l'une quelconque des revendications 8 à 11, qui est une cellule bactérienne ou une cellule de levure ou une cellule de mammifère.

**13.** Méthode de production d'une protéine pharmaceutiquement active désirée, qui comprend la culture de cellules hôtes selon l'une quelconque des revendications 8 à 12.

**14.** Protéine ayant la structure définie pu un gène de structure selon la revendication 1 ou 2.

**15.** Plasmide, qui est
le plasmide pHIRF31, FERM BP-2006;
le plasmide pIRF-L, FERM BP-2005; ou
le plasmide pIRF2-5, FERM BP-2157.

FIG .1

EP 0 359 998 B1

# FIG. 2

39

FIG. 3

Competitor (fold molar excess)

# FIG. 4A

Page a

```
  1 GGACGTGCTTTCACAGTCTAAGCCGAACCGAACCGAACCGAACCGAACCGAACCGGGCC

 60 GAGTTGCGCCGAGGTCAGCCGAGGTGGCCAGAGGACCCCAGCATCTCGGGCATCTTTCG

119 CTTCGTGCGCGCATCGCGTACCTACACCGCAACTCCGTGCCTCGCTCTCCGGCACCCTC
```

```
                                      λL28-8    1
                                      ┌─────────► Met Pro Ile Thr Arg Met Arg
178 TGCGAATCGCTCCTGCAGCAA         AGCCACC ATG CCA ATC ACT CGA ATG CGG
```

```
                                                                  20
    Met Arg Pro Trp Leu Glu Met Gln Ile Asn Ser Asn Gln Ile Pro
227 ATG AGA CCC TGG CTA GAG ATG CAG ATT AAT TCC AAC CAA ATC CCA
```

```
    Gly Leu Ile Trp Ile Asn Lys Glu Glu Met Ile Phe Gln Ile Pro
272 GGG CTG ATC TGG ATC AAT AAA GAA GAG ATG ATC TTC CAG ATT CCA
```

```
                40
    Trp Lys His Ala Ala Lys His Gly Trp Asp Ile Asn Lys Asp Ala
317 TGG AAG CAC GCT GCT AAG CAC GGC TGG GAC ATC AAC AAG GAT GCC
```

```
                                          60
    Cys Leu Phe Arg Ser Trp Ala Ile His Thr Gly Arg Tyr Lys Ala
362 TGT CTG TTC CGG AGC TGG GCC ATT CAC ACA GGC CGA TAC AAA GCA
```

```
                                                          80
    Gly Glu Lys Glu Pro Asp Pro Lys Thr Trp Lys Ala Asn Phe Arg
407 GGA GAA AAA GAG CCA GAT CCC AAG ACA TGG AAG GCA AAC TTC CGT
```

```
    Cys Ala Met Asn Ser Leu Pro Asp Ile Glu Glu Val Lys Asp Gln
452 TGT GCC ATG AAC TCC CTG CCA GAC ATC GAG GAA GTG AAG GAT CAG
```

```
            100
    Ser Arg Asn Lys Gly Ser Ser Ala Val Arg Val Tyr Arg Met Leu
497 AGT AGG AAC AAG GGC AGC TCT GCT GTG CCG GTG TAC CGG ATG CTG
```

```
                            120
    Pro Pro Leu Thr Arg Asn Gln Arg Lys Glu Arg Lys Ser Lys Ser
542 CCA CCC CTC ACC AGG AAC CAG AGG AAA GAG AGA AAG TCC AAG TCC
```

```
                                                  140
    Ser Arg Asp Thr Lys Ser Lys Thr Lys Arg Lys Leu Cys Gly Asp
587 AGC CGA GAC ACT AAG AGC AAA ACC AAG AGG AAG CTG TGT GGA GAT
```

```
    Val Ser Pro Asp Thr Phe Ser Asp Gly Leu Ser Ser Ser Thr Leu
632 GTT AGC CCG GAC ACT TTC TCT GAT GGA CTC AGC AGC TCT ACC CTA
```

# FIG. 4A

Page b

```
              160
        Pro Asp Asp His Ser Ser Tyr Thr Thr Gln Gly Tyr Leu Gly Gln
677  CCT GAT GAC CAC AGC AGT TAC ACC ACT CAG GGC TAC CTG GGT CAG

                                   180
        Asp Leu Asp Met Glu Arg Asp Ile Thr Pro Ala Leu Ser Pro Cys
722  GAC TTG GAT ATG GAA AGG GAC ATA ACT CCA GCA CTG TCA CCG TGT

                                                          200
        Val Val Ser Ser Ser Leu Ser Glu Trp His Met Gln Met Asp Ile
767  GTC GTC AGC AGC AGT CTC TCT GAG TGG CAT ATG CAG ATG GAC ATT

        Ile Pro Asp Ser Thr Thr Asp Leu Tyr Asn Leu Gln Val Ser Pro
812  ATA CCA GAT AGC ACC ACT GAT CTG TAT AAC CTA CAG GTG TCA CCC

              220
        Met Pro Ser Thr Ser Glu Ala Ala Thr Asp Glu Asp Glu Glu Gly
857  ATG CCT TCC ACC TCC GAA GCC GCA ACA GAC GAG GAT GAG GAA GGG

                                   240
        Lys Ile Ala Glu Asp Leu Met Lys Leu Phe Glu Gln Ser Glu Trp
902  AAG ATA GCC GAA GAC CTT ATG AAG CTC TTT GAA CAG TCT GAG TGG

                                                          260
        Gln Pro Thr His Ile Asp Gly Lys Gly Tyr Leu Leu Asn Glu Pro
947  CAG CCG ACA CAC ATC GAT GCC AAG GGA TAC TTG CTC AAT GAG CCA

        Gly Thr Gln Leu Ser Ser Val Tyr Gly Asp Phe Ser Cys Lys Glu
992  GGG ACC CAG CTC TCT TCT GTC TAT GGA GAC TTC AGC TGC AAA GAG

              280
        Glu Pro Glu Ile Asp Ser Pro Arg Gly Asp Ile Gly Ile Gly Ile
1037 GAA CCA GAG ATT GAC AGC CCT CGA GGG GAC ATT GGG ATA GGC ATA

                                   300
        Gln His Val Phe Thr Glu Met Lys Asn Met Asp Ser Ile Met Trp
1082 CAA CAT GTC TTC ACG GAG ATG AAG AAT ATG GAC TCC ATC ATG TGG

                                                          320
        Met Asp Ser Leu Leu Gly Asn Ser Val Arg Leu Pro Pro Ser Ile
1127 ATG GAC AGC CTG CTG GGC AAC TCT GTG AGG CTG CCG CCC TCT ATT

                                   329
        Gln Ala Ile Pro Cys Ala Pro
1172 CAG GCC ATT CCT TGT GCA CCA TAG   TTTGGGTCTCTGACCCGTTCTTGCCC

1222 TCCTGAGTGAGTTAGGCCTTGGCATCATGGTGGCTGTGATACAAAAAAAGCTAGACTCC

1281 TGTGGGCCCCTTGACACATGGCAAAGCATAGTCCCACTGCAAACAGGGGACCATCCTCC
```

42

# FIG. 4A

Page c

```
1340 TTGGGTCAGTGGGCTCTCAGGGCTTAGGAGGCAGAGTCTGAGTTTTCTTGTGAGGTGAA

1399 GCTGGCCCTGACTCCTAGGAAGATGGATTGGGGGGTCTGAGGTGTAAGGCAGAGGCCAT

1458 GGACAGGAGTCATCTTCTAGCTTTTTAAAAGCCTTGTTGCATAGAGAGGGTCTTATCGC

1517 TGGGCTGGCCCTGAGGGGAATAGACCAGCGCCCACAGAAGAGCATAGCACTGGCCCTAG

1576 AGCTGGCTCTGTACTAGGAGACAATTGCACTAAATGAGTCCTATTCCCAAAGAACTGCT

1635 GCCCTTCCCAACCGAGCCCTGGGATGGTTCCCAAGCCAGTGAAATGTGAAGGGAAAAAA

1694 AATGGGGTCCTGTGAAGGTTGGCTCCCTTAGCCTCAGAGGGAATCTGCCTCACTACCTG

1753 CTCCAGCTGTGGGGCTCAGGAAAAAAAAATGGCACTTTCTCTGTGGACTTTGCCACATT

1812 TCTGATCAGAGGTGTACACTAACATTTCTCCCCAGTCTAGGCCTTTGCATTTATTTATA

1871 TAGTGCCTTGCCTGGTGCCTGCTGTCTCCTCAGGCCTTGGCAGTCCTCAGCAGGCCCAG

1930 GGAAAAGGGGGGTTGTGAGCGCCTTGGCGTGACTCTTGACTATCTATTAGAAACGCCAC

1989 CTAACTGCTAAATGGTGTTTGGTCATGTGGTGGACCTGTGTAAATATGTATATTTGTCT

2048 TTTTATAAAAATTTAAGTTGTTTACAAAAAAAAAA 2082
```

FIG .4B

CELL LENGTH: 19
HYDROPATHY INDEX

SEQUENCE POSITION

EP 0 359 998 B1

# FIG. 5
### page a

```
      1                         25                              50
MOUSE MPITRMRMRPWLEMQINSNQIPGLIWINKEEMIFQIPWKHAAKHGWDINK
      ++++  +++x+*++++/*x*x*++*+*+*+xr  +*x**//r******+*
HUMAN MPITWMRMRPWLEMQINSNQIPGLIWINKEEMILEIPWKHAAKHGWDINK


                               75                             100
      DACLFRSWAIHTGRYKAGEKEPDPKTWKANFRCAMNSLPDIEEVKDQSRN
      *********+*+*+*+****+*+*++**++*+***+****+*+*****+++
      DACLFRSWAIHTGRYKAGEKEPDPKTWKANFRCAMNSLPDIEEVKDQSRN


                               125                            150
      KGSSAVRVYRMLPPLTRNQRKERKSKSSRDTKSKTKRKLCGDVSPDTFSD
      v********+***+*+* *********++**  +++  +++  v*+ ++*******
      KGSSAVRVYRMLPPLTKNQRKERKSKSSRDAKSKAKRLSCGDSSPDTFSD


                               175                            200
      GLSSSTLPDDHSSYTTQGYLGQDLDMERDITPALSPCVVSSSLSEWHMQM
      *+*+*****r*+*+**  *+  **-  +   ******** ++*  +  ++
      GLSSSTLPDDHSSYTVPGYM-QDLEVEQALTPALSPCAVSSTLPDWHIPV


                               225                            250
      DIIPDSTTDLYNLQVSPMPSTSEAATDEDEEGKIAEDLMKLFEQSEWPQT
      +++*  ++*+  x*******  +**  ********  **  +++  +*******
      EVVPDSTSDLYNFQVSPMPSISEATTDEDEEGKLPEDIMKLLEQSEWQPT


                               275                            300
      HIDGKGYLLNEPGTQLSSVYGDFSCKEEPEIDSPRGDIGIGIQHVFTEMK
      *********+****  +  **********+********+  ****    *  +**  *
      NVDGKGYLLNEPGVQPTSVYGDFSCKEEPEIDSPGGDIGLSLQRVFTDLK


                               329
      NMDSIMWMDSLLGNSVRLPPSIQAIPCAP
      *+*  *  *xx*  ++**  **********
      NMDAT-WLDSLLTP-VRLP-SIQAIPCAP
```

45

Figure 5

page b

The conserved amino acids are marked by asterisks. The sequences are presented with the one letter amino acid code as follows: A, alanine; C, cystein; D, aspartic acid; E, glutamic acid; F, phenylalanine; G, glycine; H, histidine; I, isoleucine; K, lysin; L, leucine; M, methionine; N, asparagine; P, proline; Q, glutamine; R, arginine; S, serine; T, threonine; V, valine; W, tryptophan and Y, tyrosine.

# FIG.6

A

B

# FIG . 7A

```
PstI
CTGCAGAAAGAGGGGGACGGTCTCGGCTTTCCAAGACAGGCAAGGGGG
-299

                        GC Box 1                    GC Box 2
CAGGGGAGTGGAGTGGAGCAAGGGGCGGGCCCGCGGTAGCCCCGGGGCGGTGGCGCGG
-251


GCCCGAGGGGGTGGGGAGCACAGCTGCCTTGTACTTCCCCTTCGCCGCTTAGCTCTAC
-193

                                        CAAT Box
AACAGCCTGATTTCCCCGAAATGATGAGGCCGAGTGGGCCAATGGGCGCGCAGGAGCG
-135

                                            Minor Cap site
                                                !!!
GCGCGGCGGGGGCGTGGCCGAGTCCGGGCCGGGGAATCCCGCTAAGTGTTTAGATTTC
-77                                                         -21

            Major Cap site                          plRF-L
                !!!
TTCGCGGCGCCGCGGACTCGCCAGTGCGCACCACTCCTTCGTCGAGGTAGGACGTGCT
-19                 +1


TTCACAGTCTAAGCCGAACCGAACCGAACCGAACCGAACCGAACCGGGCCGAGTTGCG
+39


CCGAGGTCAGCCGAGGTGGCCAGAGGACCCCAGCATCTCGGGCATCTTTCGCTTCGTG
+97


CGCGCATCGCGTACCTACACCGCAACTCCGTGCCTCGCTCTCCGGCACCCTCTGCGAA
+155
        PstI
TCGCTCCTGCAG
+213     +225
```

# FIG.7B

# FIG. 8

Page a

CGAGCCCCGCCGAACCGAGGCCACCCGGAGCCGTGCCCAGTCCACGC

CGGCCGTGCCCGGCGGCCTTAAGAACCAGGCAACCACTGCCTTCTTCCCT

CTTCCACTCGGAGTCGCGCTTCGCGCGCCCTCACTGCAGCCCCTGCGTCG

CCGGGACCCTCGCGCGCGACCAGCCGAATCGCTCCTGCAGCAGAGCCAAC


```
           10        20        30        40        50
ATGCCCATCACTTGGATGCGCATGAGACCCTGGCTAGAGATGCAGATTAA
MetProIleThrTrpMetArgMetArgProTrpLeuGluMetGlnIleAsn


           60        70        80        90       100
TTCCAACCAAATCCCGGGGCTCATCTGGATTAATAAAGAGGAGATGATCT
 SerAsnGlnIleProGlyLeuIleTrpIleAsnLysGluGluMetIleLeu


          110       120       130       140       150
TGGAGATCCCATGGAAGCATGCTGCCAAGCATGGCTGGGACATCAACAAG
 GluIleProTrpLysHisAlaAlaLysHisGlyTrpAspIleAsnLys


          160       170       180       190       200
GATGCCTGTTTGTTCCGGAGCTGGGCCATTCACACAGGCCGATACAAAGC
AspAlaCysLeuPheArgSerTrpAlaIleHisThrGlyArgTyrLysAla


          210       220       230       240       250
AGGGGAAAAGGAGCCAGATCCCAAGACGTGGAAGGCCAACTTTCGCTGTG
 GlyGluLysGluProAspProLysThrTrpLysAlaAsnPheArgCysAla


          260       270       280       290       300
CCATGAACTCCCTGCCAGATATCGAGGAGGTGAAAGACCAGAGCAGGAAC
   MetAsnSerLeuProAspIleGluGluValLysAspGlnSerArgAsn


          310       320       340       340       350
AAGGGCAGCTCAGCTGTGCGAGTGTACCGGATGCTTCCACCTCTCACCAA
LysGlySerSerAlaValArgValTyrArgMetLeuProProLeuThrLys


          360       370       380       390       400
GAACCAGAGAAAAGAAAGAAAGTCGAAGTCCAGCCGAGATGCTAAGAGCA
 AsnGlnArgLysGluArgLysSerLysSerSerArgAspAlaLysSerLys


          410       420       430       440       450
AGGCCAAGAGGAAGTCATGTGGGGATTCCAGCCCTGATACCTTCTCTGAT
  AlaLysArgLysSerCysGlyAspSerSerProAspThrPheSerAsp


          460       470       480       490       500
GGACTCAGCAGCTCCACTCTGCCTGATGACCACAGCAGCTACACAGTTCC
GlyLeuSerSerSerThrLeuProAspAspHisSerSerTyrThrValPro
```

# FIG.8

Page b

```
        510       520       530       540       550
AGGCTACATGCAGGACTTGGAGGTGGAGCAGGCCCTGACTCCAGCACTGT
 GlyTyrMetGlnAspLeuGluValGluGlnAlaLeuThrProAlaLeuSer

        560       570       580       590       600
CGCCATGTGCTGTCAGCAGCACTCTCCCCGACTGGCACATCCCAGTGGAA
  ProCysAlaValSerSerThrLeuProAspTrpHisIleProValGlu

        610       620       630       640       650
GTTGTGCCGGACAGCACCAGTGATCTGTACAACTTCCAGGTGTCACCCAT
 ValValProAspSerThrSerAspLeuTyrAsnPheGlnValSerProMet

        660       670       680       690       700
GCCCTCCATCTCTGAAGCTACAACAGATGAGGATGAGGAAGGGAAATTAC
 ProSerIleSerGluAlaThrThrAspGluAspGluGluGlyLysLeuPro

        710       720       730       740       750
CTGAGGACATCATGAAGCTCTTGGAGCAGTCGGAGTGGCAGCCAACAAAC
  GluAspIleMetLysLeuLeuGluGlnSerGluTrpGlnProThrAsn

        760       770       780       790       800
GTGGATGGGAAGGGGTACCTACTCAATGAACCTGGAGTCCAGCCCACCTC
 ValAspGlyLysGlyTyrLeuLeuAsnGluProGlyValGlnProThrSer

        810       820       830       840       850
TGTCTATGGAGACTTTAGCTGTAAGGAGGAGCCAGAAATTGACAGCCCAG
  ValTyrGlyAspPheSerCysLysGluGluProGluIleAspSerProGly

        860       870       880       890       900
GGGGGGATATTGGGCTGAGTCTACAGCGTGTCTTCACAGATCTGAAGAAC
  GlyAspIleGlyLeuSerLeuGlnArgValPheThrAspLeuLysAsn

        910       920       930       940       950
ATGGATGCCACCTGGCTGGACAGCCTGCTGACCCCAGTCCGGTTGCCCTC
 MetAspAlaThrTrpLeuAspSerLeuLeuThrProValArgLeuProSer

        960       970
CATCCAGGCCATTCCCTGTGCACCGTAGCAGGGCCCCTGGGCCCCTCTTA
  IleGlnAlaIleProCysAlaPro***


TTCCTCTAGGCAAGCAGGACCTGGCATCATGGTGGATATGGTGCAGAGAA

GCTGGACTTCTGTGGGCCCCTCAACAGCCAAGTGTGACCCCACTGCCAAG

TGGGGATGGGCCTCCCTCCTTGGGTCATTGACCTCTCAGGGCCTGGCAGG

CCAGTGTCTGGGTTTTTCTTGTGGTGTAAAGCTGGCCCTGCCTCCTGGGA

AGATGAGGTTCTGAGACCAGTGTATCAGGTCAGGGACTTGGACAGGAGTC
```

# FIG.8

Page c

AGTGTCTGGCTTTTTCCTCTGAGCCCAGCTGCCTGGAGAGGGTCTCGCTG

TCACTGGCTGGCTCCTAGGGGAACAGACCAGTGACCCCAGAAAAGCATAA

CACCAATCCCAGGGCTGGCTCTGCACTAAGAGAAAATTGCACTAAATGAA

TCTCGTTCCAAAGAACTACCCCTTTTCAGCTGAGCCCTGGGGACTGTTCC

AAAGCCAGTGAATGTGAAGGAAAGTGGGGTCCTTCGGGGCAATGCTCCCT

CAGCCTCAGAGGAGCTCTACCCTGCTCCCTGCTTTGGCTGAGGGGCTTGG

GAAAAAAACTTGGCACTTTTTCGTGTGGATCTTGCCACATTTCTGATCAG

AGGTGTACACTAACATTTCCCCCGAGCTCTTGGCCTTTGCATTTATTTAT

ACAGTGCCTTGCTCGGGGCCCACCACCCCCTCAAGCCCCAGCAGCCCTCA

ACAGGCCCAGGGAGGGAAGTGTGAGCGCCTTGGTATGACTTAAAATTGGA

AATGTCATCTAACCATTAAGTCATGTGTGAACACATAAGGACGTGTGTAA

ATATGTACATTTGTCTTTTTATAAAAAGTAAAATTGTT

# FIG.9.